# EUROPÄISCHE PATENTSCHRIFT

(11) **EP 3 267 963 B1**
(45) Veröffentlichungstag und Bekanntmachung des Hinweises auf die Patenterteilung: **12.02.2020**
(21) Anmeldenummer: 16707818.7
(22) Anmeldetag: 04.03.2016
(51) Int. Cl.: A61K 6/00, A61K 6/083

(54) **POLYMERISIERBARES DENTALMATERIAL MIT PHASENTRANSFERKATALYSATOR**
POLYMERIZABLE DENTAL MATERIAL HAVING A PHASE TRANSFER CATALYST
MATÉRIAU DENTAIRE POLYMÉRISABLE AVEC CATALYSEUR DE TRANSFERT DE PHASE

(30) Priorität: 09.03.2015 DE 102015103427
(43) Veröffentlichungstag der Anmeldung: 17.01.2018
(73) Patentinhaber: Kettenbach GmbH & CO. KG, 35713 Eschenburg (DE)
(72) Erfinder: BUBLEWITZ, Alexander, 35745 Herborn (DE); THEIS, Alexander, 35713 Eschenburg (DE)
(74) Vertreter: Keil & Schaafhausen Patent- und Rechtsanwälte PartGmbB
(86) Internationale Anmeldenummer: PCT/EP2016/054672
(87) Internationale Veröffentlichungsnummer: WO 2016/142296

(56) Entgegenhaltungen:
- WO-A2-2014/033280
- US-A1- 2012 202 913
- US-B2- 7 741 381

## Beschreibung

Die vorliegende Erfindung betrifft ein polymerisierbares Dentalmaterial, enthaltend wenigstens eine Katalysatorpaste und wenigstens eine Basispaste mit jeweils ausgewählten Komponenten.

Polymerisierbare Dentalmaterialien der vorliegenden Erfindung können bei der therapeutischen Behandlung zum Aufbau und der Wiederherstellung von zerstörter, natürlicher Zahnsubstanz verwendet werden. Insbesondere wenn noch ein Zahnstumpf vorhanden ist, welcher eine feste Verankerung des Zahnstumpfs im Kiefer sicherstellt, finden diese Dentalmaterialien bei stark zerstörten Zähnen zum Stumpfaufbau Anwendung. Beispielsweise können Zahnstümpfe von stark zerstörten Zähnen mit aufgetragenem Dentalmaterial aufgebaut werden oder große Löcher in der natürlichen Zahnsubstanz können geschlossen werden, um unter funktionalen Gesichtspunkten ein für den Patienten zufriedenstellendes Ergebnis zu erhalten. Folglich müssen die Dentalmaterialien eine ausreichend hohe Härte aufweisen. Weitere bevorzugte Anwendungsformen sind die Befestigung von prothetischen Werkstoffen wie z.B. Kronen, Brücken, Inlays, Onlays oder Veneers. Insbesondere ist für beide Anwendungen entscheidend, dass die Dentalmaterialien ausreichend auf dem noch vorhandenen Zahnmaterial haften, da die aufgetragenen Dentalmaterialien beim Kauen hohen Kräften ausgesetzt sind. Die gattungsgemäßen, polymerisierbaren Dentalmaterialien können je nach geplanter Verwendung unterschiedliche Anforderungen erfüllen.

Zum Aufbau fehlender Zahnsubstanz verwendete polymerisierbare Dentalmaterialien werden auch als Stumpfaufbaumaterialien bezeichnet. Für solche Stumpfaufbaumaterialien werden vorzugsweise hydrophobe Monomere verwendet. Um eine Applikation durch den behandelnden Zahnarzt zu erleichtern, ist es bevorzugt, dass diese Stumpfaufbaumaterialien in pastöser Form vorliegen, sodass der Zahnarzt den Zahnstumpf im Mund des Patienten aufbauen und zumindest grob modellieren kann. Um eine hohe Festigkeit und ausreichende Polymerisation der polymerisierbaren Monomere sicherzustellen, wurde bei der Verwendung von rein lichthärtenden Kompositen oftmals nur eine dünne Schicht an Komposit (polymerisierbares Dentalmaterial) auf den Zahnstumpf aufgetragen, diese mit einer Behandlungslampe ausgehärtet (also polymerisiert) und anschließend eine weitere Schicht aufgetragen. Ein solcher Schichtaufbau ist, insbesondere für rein lichthärtende Materialien unerlässlich, jedenfalls aber äußerst zeitintensiv und daher sowohl für den behandelnden Zahnarzt als auch für den Patienten nachteilhaft.

Eine weitere Anwendung polymerisierbarer Dentalmaterialien betrifft sogenannte polymerisierbare Kompositzemente. Diese werden insbesondere zur Befestigung von Prothetikwerkstoffen, wie Zahnkronen und anderen Zahnersatzmaterialien, verwendet. Schließlich kommen polymerisierbare Dentalmaterialien auch als sogenannte Bulk-Fill Komposite zur Füllung von Zahnkavitäten in Betracht.

Wird das polymerisierbare Dentalmaterial auf die natürliche Zahnsubstanz aufgetragen, so muss eine feste Verbindung zwischen aufgetragenem Komposit-Material und natürlicher Zahnsubstanz hergestellt werden. Durch die Verwendung von Adhäsiven kann eine solche Haftung erreicht werden, indem die Adhäsive auf der Zahnsubstanz zu einer besseren Benetzung und damit zu einer verbesserten Haftung des aufgetragenen Materials auf der Zahnsubstanz führen. Die Adhäsive werden vorzugsweise in flüssiger Form verwendet, damit diese einfach und gleichmäßig auf der Zahnsubstanz verteilt werden können.

Um die oben beschriebene Haftvermittlung zwischen aufgetragenen Material und natürlicher Zahnsubstanz herzustellen, werden im Wesentlichen drei Maßnahmen durchgeführt.

Zunächst wird als erste Maßnahme eine Ätzung der natürlichen Zahnsubstanz vorgenommen, welche die Oberfläche der Zahnsubstanz, insbesondere den Zahnschmelz, aufraut. In Hinblick auf die innerliegende Zahnsubstanz, insbesondere das Dentin, kann durch die Ätzung ein Herauslösen von Mineralien aus dem Kollagenverbund der natürlichen Zahnsubstanz, insbesondere des Dentins, erreicht werden. Anschließend verbleiben auf der Zahnoberfläche im Wesentlichen geöffnete Dentintubuli und freigelegte Kollagenfasern.

Für eine ausreichende Haftung zwischen aufgetragenen Dentalmaterial und natürlicher Zahnsubstanz, ist es notwendig in einer zweiten Maßnahme, die geätzte Zahnoberfläche mittels eines sogenannten Primers zu beschichten. Damit diese Beschichtung ausreichend an den hydrophilen Kollagenfasern haftet, enthält der Primer hydrophile Monomere, bspw. 2-Hydroxyethylmethacrylat (HEMA), welche den freigelegten Kollagenfaserverbund durchdringen können. Anschließend härtet der Primer aus.

Da die aufzutragenden polymerisierbaren Dentalmaterialien typischerweise hydrophobe Monomere aufweisen, würde ein unmittelbares Aufbringen des polymerisierbaren Dentalmaterials auf den hydrophilen Primer keine ausreichende Haftung zwischen beiden Materialien erreichen. Daher muss zunächst eine weitere Schicht, ein sogenanntes Bonding, als dritter Schritt aufgetragen werden, um eine ausreichende Haftung zwischen polymerisierbaren Dentalmaterial und natürlicher Zahnsubstanz zu erreichen.

Die beschriebenen Maßnahmen können über unterschiedliche, separat abgepackte und aufzubringende Substanzen in mehreren Behandlungsschritten durchgeführt werden.

Für die Ätzung (erste Maßnahme) wird üblicherweise 35-%ige Phosphorsäure verwendet, welche auf der natürlichen Zahnsubstanz aufgetragen wird und für bis zu 20 Sekunden darauf verbleibt. Anschließend wurde die Säure entfernt, indem diese mit Wasser abgespült wird. Die behandelten Stellen werden daraufhin mit Luft getrocknet. Als nächste Substanz kann auf die geätzte Zahnsubstanz ein Primer (zweite Maßnahme) aufgetragen werden. Nach dessen Auspolymerisieren, wird schließlich das Bonding (dritte Maßnahme) aufgebracht, welches ebenfalls aushärtet, also auspolymerisiert.

Nachteilhaft an dem beschriebenen Aufbringen von drei Substanzen (Phosphorsäure, Primer, Bonding) ist insbesondere die lange Behandlungszeit aufgrund der durchzuführenden Behandlungsschritte: Ätzen, Spülen mit Wasser, Trockenblasen, Primer auftragen, Primer auspolymerisieren, Bonding auftragen und Bonding auspolymerisieren. Weiterhin ist dabei nachteilhaft, dass drei unterschiedliche Substanzen in drei unterschiedlichen Verpackungen bevorratet werden müssen.

Daher wurden Produkte entwickelt, welche eine oder mehrere der oben beschriebenen Maßnahmen in einer Substanz oder einem Substanzgemisch vereinen.

In einer Variante wird zunächst die Ätzung durchgeführt und dann ein Produkt aufgebracht, welches sowohl hydrophile als auch hydrophobe Monomere umfasst und daher die Funktion des Primers und des Bondings übernimmt (sogenannte Prime + Bond Systeme).

In einer anderen Variante wird die Ätzung mit dem Primer derart kombiniert, dass an den hydrophilen Monomeren des Primers Phosphorsäuregruppen vorgesehen sind, welche die natürliche Zahnsubstanz ätzen. Nach auspolymerisieren dieses Primers mit Phosphorsäuregruppen wird das Bonding aufgetragen.

Durch beide Varianten können Behandlungsschritte eingespart werden und es sind nur noch zwei separat abgepackte Substanzen erforderlich.

Eine Weiterentwicklung der oben beschriebenen Adhäsive stellen die sogenannten All-in-One-Adhäsive, One-Step Adhäsive oder auch Ein-Schritt Haftvermittler dar. Diese umfassen die oben beschriebenen drei Maßnahmen in einem einzigen Produkt. Mit anderen Worten kombinieren diese All-in-one-Adhäsive die Ätzung, den Primer und das Bonding zu einem einzelnen Behandlungsschritt. Daher erreicht die Verwendung der All-in-one-Adhäsive eine erhebliche Zeitersparnis für den behandelnden Zahnarzt und den Patienten.

Bei der Behandlung mit All-in-One-Adhäsiven und in einigen Fällen auch mit Prime + Bond Systemen hat es sich allerdings als problematisch herausgestellt, dass die Phosphorsäuregruppen der enthaltenden Monomere nicht nur die natürliche Zahnsubstanz ätzen, sondern auch mit den aufzutragenden polymerisierbaren Dentalmaterialien reagieren können und deren Aushärtung stören.

Die aufzutragenden polymerisierbaren Dentalmaterialien müssen bereits bei moderaten Temperaturen im Mundraum des Patienten aushärten, also auspolymerisieren. Eine Erhitzung der polymerisierbaren Dentalmaterialien zur Initiierung einer Polymerisation (auf typischerweise 80 °C) wäre für den Patienten höchst unangenehm und kommt daher nicht in Betracht.

Bei der chemischen Härtung werden aus den oben genannten Gründen in der Regel Initiatoren für die Polymerisation des polymerisierbaren Dentalmaterials verwendet, die bei moderaten Temperaturen, nämlich einer Mundraumtemperatur von etwa 36 °C, schnell aushärten. Daher finden überwiegend Redoxinitiatoren Anwendung, die eine Polymerisation des Dentalmaterials auslösen. Typischerweise wird ein Redoxinitiatorensystem verwendet, welches eine Per-Sauerstoffverbindung zusammen mit einem Co-Initator, z.B. einem aromatischen Amin, umfasst. Dabei wird die Per-Sauerstoffverbindung in einer Paste des polymerisierbaren Dentalmaterials (der sogenannten Katalysatorpaste) und der Co-Initiator in einer anderen Paste des polymerisierbaren Dentalmaterials (der sogenannten Basispaste) vorgesehen. Werden die Per-Sauerstoffverbindung und der Co-Initiator durch Vermischen der Pasten zusammengebracht, findet eine Redoxreaktion statt, welche die zur Polymerisation der im Dentalmaterial enthaltenden organischen Monomere benötigten Radikale liefert. Beide Pasten werden getrennt voneinander aufbewahrt, um eine hohe Lagerstabilität zu gewährleisten.

Ein Beispiel für Dentalmaterialien mit besonders guten optischen und mechanischen Eigenschaften beschreibt die WO 2014/033 280 A1. Diese Dentalmaterialien können mit unterschiedlichen Initiatorsystemen gehärtet werden, wobei auf Barbitursäurederivaten beruhende Initiatorsysteme bevorzugt eingesetzt werden. Diese bevorzugten Initiatorsysteme umfassen eine Metallverbindung, eine (Pseudo)halogenidverbindung und ein Barbitursäurederivat. Optional können noch Photoinitatoren und/oder Co-Initiatoren sowie eine organische Peresterverbindung eingesetzt werden. Die bei diesem Initiatorsystem verwendeten (Pseudo)halogenidverbindungen können neben (Pseudo)halogenidanionen beliebige Kationen enthalten, unter anderem Ammoniumkationen.

Diese Schrift offenbart auch andere Initiatorsysteme, die in Ergänzung oder alternativ zum bevorzugten Barbituratsystem eingesetzt werden können. Dabei handelt es sich um Redoxinitiatorsysteme aus einer Kombination von anorganischem Peroxid, beispielswiese Natrium- oder Kaliumperoxodisulfat, mit Alkali- oder Erdalkalitoluolsulfinat oder mit Alkali- oder Erdalkalisulfit. Außerdem werden Redoxinitiatorsysteme offenbart, die mindestens ein Amin und mindestens ein organisches Peroxid umfassen und die optional noch Photoinitatoren und/oder Co-Initiatoren enthalten können.

Dieser Stand der Technik offenbart keine Kombination von organischem Peroxid, Amin, pulverförmigem Reduktionsmittel und Ammoniumsalz. Auch wird nicht offenbart, dass die im Redoxinitiatorsystem zusammen mit einem anorganischem Peroxid einsetzbaren Alkali- oder Erdalkalisulfite in der Form von Pulvern vorliegen. Dieser Stand der Technik offenbart auch keinen Einsatz von Phasentransferkatalysatoren in Kombination mit einem pulverförmigen Reduktionsmittel, die zusammen mit den übrigen Bestandteilen eines Redoxinitiatorsystems unter Mundbedingungen eine rasche und vollständige Härtung der Monomere bewirken.

In der zahnmedizinischen Praxis werden auch rein lichthärtende Dentalmaterialien verwendet, welche eine durch Lichtbestrahlung initiierte Polymerisation aufweisen. Dabei ist nachteilhaft, dass der behandelnde Zahnarzt diese Materialien in dünnen Schichten auftragen muss, da das eingestrahlte Licht nicht in tiefere Schichten des Dentalmaterials eindringen kann. Dadurch wird die Behandlung deutlich verlängert.

Um die Vorteile der chemischen Härtung mit denen der Lichthärtung zu kombinieren, wurden auch Materialien entwickelt, welche beide Initiatorsysteme enthalten und somit dualhärtende Eigenschaften aufweisen. Beispiele für den Stand der Technik von solchen 2-Komponenten Dentalmaterialien finden sich beispielsweise in EP 1 790 323 A1, EP 2 237 763 B1 oder EP 2 374 445 A2.

Werden All-in-one-Adhäsive und die beschriebenen polymerisierbaren Dentalmaterialien mit Redoxinitator bestehend aus Per-Sauerstoffverbindung und Co-Initiator für die Behandlung eingesetzt, kommt es jedoch regelmäßig zu einer nicht-ausreichenden Polymerisation an der Grenzschicht von Adhäsiv und polymerisierbaren Dentalmaterial. Dies wird dadurch erklärt, dass die im All-in-one-Adhäsiv enthaltenden Carbonsäure-, Phosphorsäure- oder Phosphonsäuregruppen die als Co-Initiatoren verwendeten Amine protonieren, wodurch diese in eine Ammoniumverbindung überführt werden. Dadurch wird die eigentlich gewünschte Redoxreaktion der (ursprünglichen) Amine mit der Perverbindung zur Initiierung der Polymerisationreaktion verhindert und es kommt an der Grenzschicht von Adhäsiv und polymerisierbaren Dentalmaterial zu einer nicht-ausreichenden Aushärtung des Dentalmaterials.

Diese Problematik tritt auch bei dualhärtenden Dentalmassen auf, wenn aufgrund der Schichtdicke das Licht der Behandlungslampe nicht bis zum Kavitätenboden, an dem sich die Grenzfläche zu dem Adhäsiv befindet, vordringt.

In solchen Fällen ist die Verwendung eines gängigen polymerisierbaren Dentalmateriales mit Redoxinitiator zusammen mit einem All-in-One-Adhäsiv mit einer verringerten Haftung des Dentalmaterials auf dem Adhäsiv verbunden. Der behandelnde Zahnarzt kann daher solche Adhäsive nur in Verbindung mit einem zusätzlichen Aktivator, der die Selbsthärtung übernimmt, verwenden oder muss auf andere Dentalmaterialien (bspw. ausschließlich lichthärtende Dentalmaterialien) ausweichen, welche jedoch die oben beschriebenen Nachteile, insbesondere eine längere Behandlungszeit, aufweisen.

Die US 7,741,381 B2 offenbart eine polymerisierbare Zusammensetzung, welche als dentales Restaurationsmaterial eingesetzt werden kann. Die Zusammensetzung zeichnet sich dadurch aus, dass eine Behinderung der Polymerisationsreaktion durch vorhandenen Luftsauerstoff verhindert werden kann. Dazu wird Wasser und ein Tensid der Zusammensetzung hinzugefügt, sodass sich ein Wasserfilm auf der härtenden Zusammensetzung ausbildet und dieser ein Eindringen des Luftsauerstoffs in die aushärtende Zusammensetzung verhindert. Insgesamt wird so die Festigkeit des gehärteten Materials verbessert.

Um die Haftwirkung zwischen Zahnsubstanz und polymerisierbaren Dentalmaterialien mit Redoxinitiator zu verbessern, beschreibt die EP 2 409 997 A1 eine

Zusammensetzung, welche in die Zahnsubstanz eindringt und die in der Zahnsubstanz enthaltene Feuchtigkeit nutzt, um eine beschleunigte Aushärtung des Dentalmaterials zu erreichen. Die beschriebene Zusammensetzung weist ein Monomerengemisch, ein anorganisches Peroxid, ein Reduktionsmittel und einen Polymerisationsbeschleuniger auf. Der Polymerisationsbeschleuniger wird von der Feuchtigkeit auf der Oberfläche der Zahnsubstanz aufgelöst, wodurch die Polymerisationsaushärtung an der Haftgrenzfläche und innerhalb der aushärtbaren Zusammensetzung verbessert wird. Beispiele für einen solchen Beschleuniger sind als Co-Initiatoren geeignete wasserlösliche Sulfite, aber auch Ammoniumsalze wie das Tetramethylammoniumsalz und das Tetraethylammoniumsalz der Benzolsulfonsäure. Gemäß der Beispiele der EP 2 409 997 A1, liegt der Polymerisationsbeschleuniger in der Basispaste vor.

Die EP 1 780 223 B1 beschreibt ein Haftmittel für den Dentalbereich, wobei als zusätzlicher Co-Initiator ein Polymerisationsbeschleuniger, nämlich ein wasserlösliches Sulfit, zugegeben wird. Das Sulfit wird in dem polymerisierbaren Monomer dispergiert und an der Grenzfläche zu der natürlichen Zahnsubstanz durch Feuchtigkeit im Zahn aufgelöst. Aufgrund der zusätzlichen Reduktionswirkung des Sulfits kommt es zu einer beschleunigten Aushärtung des polymerisierbaren Dentalmateriales an der Grenzfläche zwischen dem Zahn und dem polymerisierbaren Dentalmaterial, welches die Haftwirkung verbessert. Die Verwendung von Phasentransferkatalysatoren wird hier jedoch nicht erwähnt.

Die EP 2 554 154 A1 beschreibt ein Kit aus einem Adhäsiv und einer härtbaren Zusammensetzung mit einer hohen Haftung und Beständigkeit für den Dentalbereich. Im Gegensatz zu EP 1 780 223 B1, bei der das wasserlösliche Reduktionsmittel (Natriumsulfit) ausschließlich in dentalen Haftmitteln eingesetzt wird, die hydrophile Monomere (HEMA) enthalten, kommen in der EP 2 554 154 A1 hydrophobe Monomermischungen zum Einsatz. Das in EP 2 554 154 A1 beschriebene härtbare Komposit ist daher im Gegensatz zu den Produkten aus EP 1 780 223 B1 für die Anwendung als Stumpfaufbaumaterial geeignet. Das zugehörige Adhäsiv des oben genannten Kits umfasst eine Monomerenmischung, Wasser und ein aminbasierendes, schwefelfreies Reduktionsmittel. Das Stumpfaufbaumaterial umfasst ebenfalls eine Monomerenmischung, ein wasserlösliches, schwefelhaltiges Reduktionsmittel, ein organisches Peroxid und ein aminbasierendes, schwefelfreies Reduktionsmittel. Auch hier ist die Verwendung von Phasentransferkatalysatoren nicht beschrieben.

Nachteilig an der in EP 2 554 154 A1 beschriebenen Lösung ist, dass für die Anwendung des Stumpfaufbaumaterials ein spezielles in dem Kit mit geliefertes Adhäsiv erforderlich ist, welches ein zusätzliches Amin als Reduktionsmittel enthält, das die Ätzwirkung des ebenfalls in dem Adhäsiv enthaltenen Phosphorsäuremethacrylates (z.B. MDP) auf der Zahnoberfläche durch eine partielle Neutralisation verringert.

Es ist davon ausgehend Aufgabe der vorliegenden Erfindung, ein polymerisierbares Dentalmaterial bereitzustellen, das mit allen gängigen Adhäsiven, insbesondere den Prime + Bond Systemen und All-in-One-Adhäsiven, verwendet werden kann, ohne dass die oben beschriebenen Nachteile auftreten. Insbesondere ist es Aufgabe der Erfindung ein polymerisierbares Dentalmaterial bereitzustellen, welches an der Grenzfläche zu allen Adhäsiven, insbesondere den Prime + Bond Systemen und den All-in-One-Adhäsiven, eine ausreichende Aushärtung und damit einen verbesserten Haftverbund bei gleichzeitig hoher Lagerstabilität der pastösen Massen (> 1 Jahr, vorzugsweise > 2 Jahre) erreicht.

Diese Aufgabe wird mit den Merkmalen des Anspruchs 1 dadurch gelöst, dass das polymerisierbare Dentalmaterial eine Katalysatorpaste (A) und eine Basispaste (B) enthält. Die Katalysatorpaste umfasst wenigstens eine organische Per-Sauerstoffverbindung, wenigstens einen Füllstoff und wenigstens ein radikalisch polymerisierbares organisches (Meth)acrylmonomer. Die Basispaste enthält wenigstens ein radikalisch polymerisierbares organisches (Meth)acrylmonomer, wenigstens einen Füllstoff, ein Amin als Co-Initiator der radikalischen Polymerisation und wenigstens ein in der Basispaste (B) dispergiertes salzartiges, wasserlösliches und pulverförmiges Reduktionsmittel (im Folgenden auch: Reduktionsmittel). Weiterhin ist in wenigstens einer Katalysatorpaste und/oder Basispaste wenigstens ein Phasentransferkatalysator vorgesehen, welcher ein Ammonium-, ein Phosphonium- und/oder ein Sulfoniumsalz ist, das ein anorganisches oder organisches Anion enthält mit der Massgabe, dass der Phasentransferkatalysator im Fall von organischen Anionen nur solche mit 1-4 Kohlenstoffatomen aufweist und dass Anionen der Sulfinsäuren ausgenommen sind.

Durch die erfindungsgemäße Verwendung eines Phasentransferkatalysators wird somit indirekt die Haftung zwischen dem ausgehärteten Dentalmaterial und dem Adhäsiv verbessert. Dies lässt sich dadurch erklären, dass das salzartige, wasserlösliche und pulverförmige Reduktionsmittel an der Grenzfläche zum Adhäsiv aufgelöst wird und dann durch den Phasentransferkatalysator in die organische (Meth)acrylmonomer-Masse eingetragen werden kann, um auch dort eine Beschleunigung der Polymerisation durch Reaktion mit der Per-Sauerstoffverbindung zu erreichen. Aufgrund der Aufteilung des polymerisierbaren Dentalmaterials in wenigstens eine Katalysatorpaste und wenigstens eine Basispaste wird eine hohe Lagerstabilität erreicht. Durch das Vermischen dieser einzelnen Komponenten wird das polymerisierbare Dentalmaterial erzeugt, das unter Mundbedingungen (entsprechende Temperatur und Feuchtigkeit) rasch aushärtet.

Vorzugsweise ist der Anteil des Phasentransferkatalysators in der Katalysatorpaste und/ oder der Basispaste, bezogen auf die Gesamtmasse der Katalysatorpaste und/ oder der Basispaste, 0,01 bis 5 Gew.-%, bevorzugt 0,01 bis 2 Gew.-%, besonders bevorzugt 0,05 bis 1 Gew.-% und ganz besonders bevorzugt 0,05 bis 0,5 Gew.-%.

Der genannte Gewichtsanteil des Phasentransferkatalysators in der Katalysatorpaste und/ oder der Basispaste bezogen auf die Gesamtmasse der Katalysatorpaste und/ oder der Basispaste muss so niedrig gewählt werden, dass die Aushärtung des polymerisierbaren Dentalmaterials an der Grenzfläche zum Adhäsiv nicht derart schnell verläuft, dass eine Verarbeitung des Dentalmaterials erschwert oder gar verhindert wird. Dies ist insbesondere dann wichtig, wenn das Produkt auch im Wurzelkanal angewendet werden kann, da ansonsten das Einsetzen des Wurzelstiftes aufgrund einer vorzeitigen Verblockung des Wurzelkanals unmöglich werden kann. Gleichzeitig sollte der genannte Gewichtsanteil derart hoch gewählt werden, dass die Polymerisation an der Grenzfläche zu dem Adhäsiv in etwa gleich schnell wie die Polymerisation innerhalb der Dentalmaterial-Masse erfolgt, um ein Ablösen der ansonsten gehärteten Masse von der Zahnsubstanz zu vermeiden.

Es hat sich herausgestellt, dass es in Hinblick auf die Lagerstabilität des polymerisierbaren Dentalmaterials von Vorteil ist, den Phasentransferkatalysator nur in der wenigstens einen Katalysatorpaste vorzusehen. Dies wird damit erklärt, dass Reste von Feuchtigkeit in den Pasten nicht auszuschließen sind und insbesondere bei Lagerung der Pasten Feuchtigkeit in deren Verpackungen eindringen kann. Diese Feuchtigkeit kann das in der Basispaste dispergierte Reduktionsmittel auflösen und dieses dadurch für Redoxreaktionen aktivieren. Sollte der Phasentransferkatalysator bereits zusammen mit dem Reduktionsmittel in einer Basispaste vorliegen, so ist nicht auszuschließen, dass es im Zusammenhang mit vorhandener Feuchtigkeit zu einem verstärktem Eintragen des Reduktionsmittels in das organische (Meth)acrylmonomer kommt, und eine Reaktion zwischen dem Reduktionsmittel und dem Monomer stattfindet. Auch kann in der Basispaste eingeschlossener Luftsauerstoff als Oxidationsmittel mit dem Reduktionsmittel reagieren und dadurch eine Polymerisation des organischen (Meth)acrylmonomer während der Lagerung auslösen. Damit würde die Basispaste unbrauchbar.

Ist der Phasentransferkatalysator in der Basispaste vorgesehen, so ist es von Vorteil Maßnahmen zu treffen, welche eine vorzeitige Umsetzung von Reduktionsmittel, Phasentransferkatalysator und dem (Meth)acrylmonomer verhindern, um die Lagerstabilität der Basispaste zu erhöhen. Dazu hat es sich als vorteilhaft herausgestellt, wenn der Basispaste ein Trocknungsmittel zugegeben wird, welches darin vorhandenes oder während der Lagerung in die Basispaste eindringendes Wasser bindet und dadurch ein Lösen des Reduktionsmittels verhindert. Alternativ oder ergänzend dazu kann der Basispaste eine Base zugesetzt werden, welche eine Redoxreaktion des Reduktionsmittels mit Luftsauerstoff verhindert oder zumindest verlangsamt.

Beispiele für Trocknungsmittel umfassen: Silicagel, Zeolithe, Aluminiumoxid, Calciumoxid und/oder Calciumsulfat sowie Magnesiumoxid und/oder Magnesiumsulfat.

In einer bevorzugten Ausführungsform der Erfindung ist der Phasentransferkatalysator in der Katalysatorpaste enthalten. Dies hat sich in Hinblick auf die Lagerstabilität als besonders günstig herausgestellt, da eine potentielle Aktivierung des in der Basispaste vorhandenen Reduktionsmittels mit dem Phasentransferkatalysator und eine anschließende Reaktion des Reduktionsmittels mit Luftsauerstoff wie oben beschreiben dadurch verhindert werden kann.

Als Reduktionsmittel wird erfindungsgemäß eine Substanz verstanden, die in Kombination mit der Per-Sauerstoffverbindung ein Redoxsystem bildet, das zur Initiierung einer radikalischen Polymerisation des polymerisierbaren (Meth)acrylmonomers geeignet ist. Weiterhin kann das Reduktionsmittel eine Reaktion einer radikalischen, wachsenden Polymerkette mit dem Diradikal Sauerstoff verhindern, was zum Abbruch der Polymerisationsreaktion führen kann, indem das Reduktionsmittel nach dem Auflösen mit dem Sauerstoff reagiert.

Als wasserlöslich im Sinne der vorliegenden Erfindung wird eine Substanz verstanden, welche eine Löslichkeit von mindestens 10 g/L, vorzugsweise mindestens 15 g/L, besonders bevorzugt mindestens 30 g/L, und ganz besonders bevorzugt mindestens 50 g/L in destilliertem Wasser bei einer Temperatur von 25 °C aufweist.

Vorzugsweise ist das Reduktionsmittel ausgewählt aus der Gruppe der Sulfite, insbesondere aus der Gruppe der Alkalimetallsulfite, Erdalkalimetallsulfite, (NH₄)₂SO₃, oder der Hydrogensulfite (Bisulfite), Disulfite, Thiosulfite, Thionate und Dithionite, insbesondere der Alkalimetallsalze oder der Erdalkalimetallsalze davon. Besonders bevorzugt wird als Reduktionsmittel Natriumsulfit eingesetzt. Die genannten Reduktionsmittel haben sich als besonders geeignet für die vorliegende Erfindung herausgestellt, da diese eine geringe Löslichkeit in dem organischen Monomer aufweisen und in der Basispaste dispergiert vorliegen, aber besonders gut von dem erfindungsgemäß eingesetzten Phasentransferkatalysator in das organische Monomer eingetragen werden können.

Der Gewichtsanteil des Reduktionsmittels, bezogen auf die Gesamtmasse der wenigstens einen Basispaste, beträgt vorzugsweise weniger als 10 Gew.-%, besonders bevorzugt weniger als 5 Gew.-%, und ganz besonders bevorzugt 2±1 Gew.-%. Eine zu hohe Menge an Reduktionsmittel führt zu einer zu hohen Löslichkeit der polymerisierbaren Masse, so dass nach der Aushärtung durch Auswaschungen mit Speichel ein poröses Produkt entstehen kann, bei dem die mechanische Festigkeit deutlich reduziert ist. Gleichzeitig muss der genannte Gewichtsanteil derart hoch gewählt werden, dass die Polymerisation des Dentalmaterials wie beabsichtigt auch an der Grenzfläche zu dem Adhäsiv stattfindet.

In der wenigstens einen Basispaste ist ein Amin als Co-Initiator vorgesehen, welches unabhängig vom Reduktionsmittel zur Initiierung einer Polymerisationsreaktion des organischen (Meth)acrylmonomer mit der Per-Sauerstoff-Verbindung geeignet ist.

In der Regel wird der Co-Initiator ausgewählt aus der Gruppe der primären, sekundären und/oder tertiären Amine, insbesondere der sekundären Amine und/oder der tertiären Amine. Beispiele geeigneter sekundärer Amine und/ oder geeigneter tertiärer Amine umfassen o-Tolyldiethanolamin, m-Tolyldiethanolamin, p-Tolyldiethanolamin, N-Methylanilin, N-Methyl-o-toluidin, N-Methyl-m-toluidin, N-Methyl-p-toluidin, Methyl-2-anisidin, Methyl-3-anisidin, Methyl-4-anisidin, N,N,-Dimethyl-o-toluidin, N,N,-Dimethyl-m-toluidin und/oder N,N,-Dimethyl-p-toluidin.

Der Gewichtsanteil des eingesetzten Co-Initiators, bezogen auf die Gesamtmasse der wenigstens einen Basispaste, beträgt vorzugsweise weniger als 5 Gew.-%, besonders bevorzugt weniger als 2 Gew.-%, und ganz besonders bevorzugt 1±0,5 Gew.-%.

Vorzugsweise ist die Per-Sauerstoffverbindung ein Peroxid, ein Peroxidester, insbesondere ein Diacylperoxid, ein Dialkylperoxid, ein Peroxyketal, ein Peroxyketon oder ein Hydroperoxid. Entscheidend für die eingesetzte Per-Sauerstoff-Verbindung ist, dass die Per-Sauerstoff-Verbindung in Kombination mit dem Reduktionsmittel und dem Co-Initiator ein Redoxsystem bildet, das zur Initiierung einer radikalischen Polymerisation des organischen (Meth)acrylmonomer geeignet ist. Mit anderen Worten müssen die Redoxpotentiale der Per-Sauerstoff-Verbindung und des Reduktionsmittels bzw. des Co-Initiators derart aufeinander abgestimmt sein, dass es zu einer Redoxreaktion zwischen beiden kommt und, dass dabei Radikale gebildet werden, welche eine Polymerisation des (Meth)acrylmonomer starten.

In einer bevorzugten Ausführungsform ist die organische Per-Sauerstoff-Verbindung ein Diacylperoxid, insbesondere ein Benzoylperoxid oder dessen halogenierte Derivate, bevorzugt Dibenzoylperoxid.

Der Gewichtsanteil der Per-Sauerstoffverbindung, bezogen auf die Gesamtmasse der wenigstens einen Katalysatorpaste, beträgt vorzugsweise weniger als 5 Gew.-%, besonders bevorzugt weniger als 2 Gew.-%, besonders bevorzugt weniger als 1 Gew.-% und ganz besonders bevorzugt 0,8±0,2 Gew.-%.

Die genannte Redoxinitiatorsysteme können auch mit mindestens einem weiteren Initiatorsystem ergänzt werden. So kann bspw. mindestens ein Photoinitiator und/oder mindestens ein thermisch aktivierbarer Radikalstarter, beispielsweise Azoverbindungen, in dem polymerisierbaren Dentalmaterial vorgesehen sein. Diese zusätzlichen Initiatorsysteme können in der wenigstens einen Katalysatorpaste und/oder der wenigstens einen Basispaste vorgesehen sein. Dem Fachmann sind derartige Initiatorklassen bekannt.

Ein vorzugsweise vorgesehener Photoinitiator ermöglicht dem Behandler jederzeit eine vorzeitige Aushärtung mit einer Polymerisationslampe. Üblicherweise werden zur Lichthärtung von Dentalmaterialien Photoinitiatorsysteme aus Campherchinon und einem aromatischen Amin eingesetzt. Eine solche Mischung erzeugt bei der Bestrahlung mit blauem Licht mit einer Wellenlänge im Bereich von 470 nm Radikale, die in weniger als einer Minute das Material vollständig härten. Der Photoinitiator kann in der Katalysatorpaste und/oder der Basispaste vorliegen.

Als Photoinitiatoren eignen sich beispielsweise alpha-Diketone wie Campherchinon, insbesondere D,L-Campherchinon, in Verbindung mit sekundären und tertiären Aminen wie z.B. Ethyl-4-dimethylaminobenzoat oder (2-Ethylhexyl)-4-dimethylaminobenzoat, und ggfs. Mono- und Bisacylphosphinoxide, wie 2,4,6-Trimethyl-benzoyldiphenylphosphinoxid und Bis-(2,6-dichlorbenzoyl)-4-n-propylphenyl-phosphinoxid und Benzaldehyde.

Der Gewichtsanteil der eingesetzten Photoinitiator-Komponenten, bezogen auf die Gesamtmasse der wenigstens einen Basispaste oder der wenigstens einen Katalysatorpaste, beträgt vorzugsweise weniger als 10 Gew.-%, bevorzugt weniger als 5 Gew.-%, besonders bevorzugt weniger als 2 Gew.-% und ganz besonders bevorzugt weniger als 1 Gew.-%.

Insbesondere für die Verwendung des polymerisierbaren Dentalmaterials als Stumpfaufbaumaterial und als polymerisierbarer Kompositzement ist es bevorzugt, wenn sowohl ein Redoxinitiatorsystem als auch ein Photoinitiator vorgesehen ist. Solche polymerisierbaren Dentalmaterialien werden auch als dualhärtend bezeichnet.

Das (Meth)acrylmonomer wird in einer bevorzugten Ausgestaltung der Erfindung ausgewählt aus der Gruppe der Acrylate bzw. Methacrylate (zusammenfassend (Meth)acrylate genannt) und/oder der Acrylamide bzw. Methacrylamide (zusammenfassend (Meth)acrylamide genannt). Dabei können sowohl bi- oder höherfunktionelle Acrylsäure- und Methacrylsäureverbindungen, wie (Meth)acryl-säureester als auch monofunktionelle (Meth)acrylsäure-verbindungen, wie (Meth)acrylsäureester, vorgesehen werden.

Bei den (Meth)acrylmonomeren im Sinne der vorliegenden Erfindung kann es sich um Einzelverbindungen, um Oligomere und/oder um Polymere handeln, die mindestens eine polymerisierbare von (Meth)acrylsäure abgeleitete Gruppe, wie eine (Meth)acrylat- oder eine (Meth)acrylamidgruppe, enthalten.

Unter Einzelverbindungen sind im Rahmen der vorliegenden Erfindung Verbindungen zu verstehen, die keine wiederkehrenden Struktureinheiten aufweisen.

Unter Oligomeren sind im Rahmen der vorliegenden Erfindung Verbindungen zu verstehen, die zwei bis zehn wiederkehrende Struktureinheiten aufweisen.

Unter Polymeren sind im Rahmen der vorliegenden Erfindung Verbindungen zu verstehen, die mehr als zehn wiederkehrende Struktureinheiten aufweisen.

Zu den bevorzugt eingesetzten radikalisch polymerisierbaren organischen (Meth)acrylmonomern zählen aromatische Gruppen enthaltende Acrylate oder Methacrylate, aliphatische Gruppen enthaltende Acrylate oder Methacrylate, Oligo- und Polyethergruppen enthaltende Acrylate oder Methacrylate, Oligo- und Polyestergruppen enthaltende Acrylate oder Methacrylate, Urethangruppen enthaltende Acrylate oder Methacrylate oder Kombinationen von zwei oder mehreren dieser Monomere.

Beispiele geeigneter (Meth)acrylate umfassen Bisphenol-A-di(meth)acrylat, BisGMA (ein Additionsprodukt aus Methacrylsäure und Bisphenol-A-diglycidether), ethoxyliertes Bisphenol-A-di(meth)acrylat, UDMA (ein Isomerengemisch aus Di-2-methacryloxyethyl-2,2,4-trimethylhexamethylendicarbamat und Di-2-(meth)-acryloxyethyl-2,4,4-trimethylhexamethylendicarbamat), 2-Hydroxyethylmethacrylat (HEMA) und/oder Gylcerin-1,3-dimethacrylat (GDMA), sowie Ethyl(meth)acrylat, Isopropyl(meth)acrylat, n-Butyl-(meth)-acrylat, Isobutyl(meth)acrylat, Tetrahydrofurfuryl(meth)acrylat, Glycidyl(meth)-acrylat, 2-Methoxyethyl-(meth)acrylat, 2-Ethoxyethyl(meth)-acrylat, 2-Methoxy-ethyl(meth)acrylat, 2-Ethylhexyl(meth)-acrylat, 2-Hydroxy-1,3-di(meth)-acryloxypropan, Neopentylglykoldi(meth)acrylat, 1,3-Butandiol-di(meth)acrylat, 1,4-Butandioldi(meth)acrylat, 1,6-Hexandioldi(meth)acrylat, 1,8-Oktandiol-di-(meth)acrylat, 1,10-Decandioldi(meth)acrylat, 1,12-Dodecandioldi(meth)acrylat, 1,14-Tetradecandioldi(meth)acrylat, 1,16-Hexadecandioldi(meth)acrylat, Trimethylolpropantri(meth)acrylat, Trimethylolethantri(meth)acrylat, Pentaerythritoltri(meth)acrylat, Dipentaerythritolpenta(meth)-acrylat, Dipentaerythritolhexa(meth)acrylat, Trimethylolmethantri(meth)acrylat, Pentaerythritoltetra(meth)acrylat, Tetraethylenglykoldi(meth)acrylat, Mono-, Oligo-, oder Polyethylenglykoldi(meth)acrylat, z.B. Ethylenglykoldi(meth)acrylat, Diethylenglykoldi(meth)acrylat und Triethylenglykoldi(meth)acrylat, Mono-, Oligo-, oder Polypropylenglykoldi(meth)acrylat, und Mono- oder Polybutylenglykol-di(meth)acrylat, insbesondere Mono- oder Polytetramethylenglykoldi(meth)acrylat, wobei bei den Polyalkylenglykolderivaten sowohl solche mit verzweigter als auch mit linearer Struktur umfasst sind.

Zudem sind (Meth)acryle mit Urethan-Bindung(en) als Beispiele für Mischungskomponenten für die genannten (Meth)acryle mit eingeschlossen. Geeignete Beispiele umfassen Di-2-(meth)acryloxyethyl-2,2',4-trimethylhexamethylendicarbamat, Di-2-(meth)acryl-oxyethyl-2,4,4'-trimethylhexamethylen-dicarbamat und 1,3,5-tris[1,3-bis{(meth)-acryloyloxy}-2-propoxycarbonylamino-hexan]-1,3,5-(1H,3H,5H)triazin-2,4,6-trion. Daneben wird exemplarisch ein (Meth)acrylat eines Urethanoligomers genannt, das sich von 2,2'-Di(4-hydroxy-cyclohexyl)propan, 2-Oxepanon, Hexamethylendiisocyanat und 2-Hydroxy-ethyl(meth)acrylat ableitet, und ein (Meth)acrylat eines Urethanoligomers, das sich von 1,3-Butandiol, Hexamethylendiisocyanat und 2-Hydroxy-ethyl(meth)-acrylat ableitet. Diese (Meth)acrylate können alleine oder als eine Mischung von zwei oder mehr in Kombination in dem polymerisierbaren Dentalmaterial verwendet werden.

Ganz besonders bevorzugt werden radikalisch polymerisierbare organische (Meth)acrylmonomere eingesetzt, die frei von Struktureinheiten mit aromatischen Resten sind, insbesondere solche radikalisch polymerisierbare organische (Meth)acrylmonomerer, die keine Struktureinheiten enthalten, die von Bisphenol A abgeleitet sind.

Erfindungsgemäß wird das (Meth)acrylmonomer in der Basispaste (B) und in der Katalysatorpaste (A) eingesetzt.

Der Phasentransferkatalysator ist ausgewählt aus der Gruppe der Ammoniumsalze, der Phosphoniumsalze und/oder der Sulfoniumsalze mit anorganischen oder organischen Anionen, wobei der Phasentransferkatalysator im Fall von organischen Anionen nur solche mit 1-4 Kohlenstoffatomen aufweist und wobei Anionen der Sulfinsäuren ausgenommen sind. Die Salze können sowohl wasserhaltig als auch wasserfrei eingesetzt werden.

Es ist weiterhin bevorzugt, wenn das Kation des Ammoniumsalzes NR₁R₂R₃R₄ ist, wobei R₁, R₂, R₃ und R₄ unabhängig voneinander C₁ bis C₂₀-, insbesondere C₁ bis C₁₀-, bevorzugt C₄-Alkyl, C₁ bis C₂₀-, insbesondere C₁ bis C₁₀-, bevorzugt C₄-Alkylhalogenid, C₁ bis C₂₀-, insbesondere C₁ bis C₁₀-, bevorzugt C₄-Alkyloxy, insbesondere Benzethonium, C₂ bis C₂₀-, insbesondere C₂ bis C₁₀-, bevorzugt C₄-Alkenyl, C₂ bis C₂₀-, insbesondere C₂ bis C₁₀-, bevorzugt C₄-Alkenyloxy, C₂ bis C₂₀-, insbesondere C₂ bis C₁₀-, bevorzugt C₄-Alkinyl, C₂ bis C₂₀-, insbesondere C₂ bis C₁₀-, bevorzugt C₄-Alkinyloxy, C₂ bis C₂₀-, insbesondere C₂ bis C₄-Alkylester, besonders bevorzugt Methylester, Aryl, vorzugsweise Phenyl, Aryloxy, Aralkyl, vorzugsweise Benzyl, Aralkyloxy, Alkylaryl, Alkylaryloxy bedeuten, wobei vorzugsweise mindestens einer der Reste R₁, R₂, R₃ oder R₄ mindestens ein C₁ Rest, vorzugsweise mindestens ein C₂ Rest, besonders bevorzugt mindestens ein C₃ Rest, und ganz besonders bevorzugt mindestens ein C₄ Rest ist. Die genannten Reste umfassen auch mögliche Konstitutions- und Stereoisomere. Ammoniumsalze mit mindestens einem C₄ Rest sind besonders bei hydrophoben Stumpfaufbaumaterialien bevorzugt.

Mehrere der genannten Reste können auch in einer besonderen Ausgestaltung der Erfindung chemisch verbunden vorliegen. Dadurch wird das Stickstoffatom des Ammoniumsalzes in einer cyclischen Verbindung, nämlich einem Heterocyclus, eingefügt. Beispiele für den Grundkörper geeigneter Heterocylen umfasen Aziridine, Azetidine, Azolidine, Azinane, Azepane, Azirine, Azete, Azole, Azine, Azepine, Pyrazole, Imidazole, Benzimidazole, Imidazoline, Indole, Chinoline, Isochinoline, Purine, Pyrimidine oder Oxazole. Diese können als entsprechend substituierte Verbindungen als Ammoniumsalze Anwendung finden. Dabei ist zu beachten, dass primäre, sekundäre und ternäre Amine bei entsprechendem Redoxpotential mit der Per-Sauerstoffverbindung reagieren können. Daher ist es bevorzugt, von den oben genannten Heterocyclen nur Verbindungen einzusetzen, die keine primäre, sekundäre und ternäre Amine enthalten.

Geeignete Beispiele für heterocyclische Ammoniumsalze umfassen N-(Allyloxycarbonyloxy)succinimid, 3-Benzyl-5-(2-hydroxyethyl)-4-methylthiazoliumchlorid, 1-Butyl-2,3-dimethylimidazoliumchlorid, 1-Butyl-2,3-dimethylimidazoliumhexafluorophosphat, 1-Butyl-2,3-dimethylimidazolium-tetrafluoroborat, 1,3-Didecyl-2-methylimidazoliumchlorid, 1-Ethyl-2,3-dimethylimidazolium-ethylsulfat, 3-Ethyl-5-(2-hydroxyethyl)-4-methylthiazolium-bromid, Hexacylpyridiniumbromid, Hexadecylpyridiniumchlorid, 5-(2-Hydroxyethyl)-3,4-dimethylthiazoliumiodid, 1-Methylimidazoliumhydrogensulfat, Methylviologendichlorid und 1,2,3-Trimethylimidazolium Salze.

Auch kommerziell erhältliche Phasentransferkatalysatoren können mit der vorliegenden Erfindung eingesetzt werden. Geeignete Beispiele umfassen Aliquat® 336, ein quarternäres Ammoniumsalz, wobei R₁ Methyl ist und R₂, R₃ und R₄ Octyl und/oder Decyl ist, wobei vor allem Octyl vorkommt, oder Arquad® 2HT-75.

Besonders bevorzugte erfindungsgemäß eingesetzte Phasentransferkatalysatoren sind Ammoniumsalze.

Ein bevorzugtes Kation des Phosphoniumsalzes ist PR₁R₂R₃,R₄ wobei R₁, R₂, R₃ und R₄ unabhängig voneinander C₁ bis C₂₀-, insbesondere C₁ bis C₁₀-, bevorzugt C₄-Alkyl, C₁ bis C₂₀-, insbesondere C₁ bis C₁₀-, bevorzugt C₄-Alkylhalogenid, C₁ bis C₂₀-, insbesondere C₁ bis C₁₀-, bevorzugt C₄-Alkyloxy, insbesondere Benzethonium, C₂ bis C₂₀-, insbesondere C₂ bis C₁₀-, bevorzugt C₄- Alkenyl, C₂ bis C₂₀-, insbesondere C₂ bis C₁₀-, bevorzugt C₄-Alkenyloxy, C₂ bis C₂₀-, insbesondere C₂ bis C₁₀-, bevorzugt C₄-Alkinyl, C₂ bis C₂₀-, insbesondere C₂ bis C₁₀-, bevorzugt C₄-Alkinyloxy, C₂ bis C₂₀-, insbesondere C₂ bis C₄-Alkylester, besonders bevorzugt Methylester, Aryl, vorzugsweise Phenyl, Aryloxy, Aralkyl, vorzugsweise Benzyl, Aralkyloxy, Alkylaryl, Alkylaryloxy bedeuten, wobei vorzugsweise mindestens einer der Reste R₁, R₂, R₃ oder R₄ mindestens ein C₁ Rest, vorzugsweise mindestens ein C₂ Rest, besonders bevorzugt mindestens ein C₃ Rest, und ganz besonders bevorzugt mindestens ein C₄ Rest ist.

Ein bevorzugtes Kation des Sulfoniumsalzes ist SR₁R₂R₃, wobei R₁, R₂ und R₃ unabhängig voneinander C₁ bis C₂₀-, insbesondere C₁ bis C₁₀-, bevorzugt C₄-Alkyl, C₁ bis C₂₀-, insbesondere C₁ bis C₁₀-, bevorzugt C₄-Alkylhalogenid, C₁ bis C₂₀-, insbesondere C₁ bis C₁₀-, bevorzugt C₄-Alkyloxy, insbesondere Benzethonium, C₂ bis C₂₀-, insbesondere C₂ bis C₁₀-, bevorzugt C₄-Alkenyl, C₂ bis C₂₀-, insbesondere C₂ bis C₁₀-, bevorzugt C₄-Alkenyloxy, C₂ bis C₂₀-, insbesondere C₂ bis C₁₀-, bevorzugt C₄-Alkinyl, C₂ bis C₂₀-, insbesondere C₂ bis C₁₀-, bevorzugt C₄-Alkinyloxy, C₂ bis C₂₀-, insbesondere C₂ bis C₄-Alkylester, besonders bevorzugt Methylester, Aryl, vorzugsweise Phenyl, Aryloxy, Aralkyl, vorzugsweise Benzyl, Aralkyloxy, Alkylaryl, Alkylaryloxy bedeuten, wobei vorzugsweise mindestens einer der Reste R₁, R₂ oder R₃ mindestens ein C₁ Rest, vorzugsweise mindestens ein C₂ Rest, besonders bevorzugt mindestens ein C₃ Rest, und ganz besonders bevorzugt mindestens ein C₄ Rest ist.

Das Anion des Ammoniumsalzes, des Phosphoniumsalzes und/oder des Sulfoniumsalzes ist ein anorganisches oder ein ausgewähltes organisches Anion mit 1-4 Kohlenstoffatomen und mit Ausnahme eines Anions der Sulfinsäure. Letztere Anionen weisen eine zu große Redox-Aktivität auf und führen zu Dentalmassen mit einer nicht ausreichenden Lagerstabilität. Organische Anionen mit 1-4 Kohlenstoffatomen verleihen dem Phasentransferkatalysator eine ausreichend hohe Hydrophilie, so dass ein ausreichend schneller Übergang in die an der Grenzfläche zum Adhäsiv existierende hydrophile Phase aus aufgelöstem Reduktionsmittel möglich ist und nach dem Eintritt in diese Phase auch der Austausch des Anions gegen das hydrophile reduzierende Anion begünstigt ist, welches dann wieder in die organische Phase transportiert werden muss.

Besonders bevorzugt erfindungsgemäß eingesetzte Phasentransferkatalysatoren sind solche, die keine Anionen von Sulfonsäuren enthalten.

Ganz besonders bevorzugt erfindungsgemäß eingesetzte Phasentransferkatalysatoren sind solche, die anorganische Anionen enthalten.

Beispiele für bevorzugte Anionen sind Anionen ausgewählt aus der Gruppe der Halogenide, der Hydroxide, Anionen von anorganischen Säuren, Pseudohalogenanionen oder Halogenkomplexe von Aluminat, Borat, Silikat oder Phosphat, oder Anionen von kurzkettigen, organischen Säuren mit 1 bis 4 Kohlenstoffatomen, beispielsweise von Carbonsäuren mit ein bis vier Kohlenstoffatomen, ausgenommen Anionen von Sulfinsäuren,
Besonders bevorzugte Anionen sind Fluorid, Chlorid, Bromid, lodid, Hydroxid, Sulfat, Hydrogensulfat, Phosphat, Hydrogenphosphat, Dihydrogenphosphat, Phosphonat, Borat, Chlorat, Perchlorat, Nitrit, Nitrat, Hydrogencarbonat, Carbonat, Tetrafluoroborat, Tetrachloroaluminat, Hexafluorosilikat, Hexachlorophosphat, Formiat, Acetat, Butyrat, Fumarat, Maleat, Glutarat, Lactat, Malat, Malonat, Oxalat, Pyruvat oder Tartrat.

Als besonders günstig haben sich die folgenden Anionen herausgestellt: Hydrogensulfat, Sulfat, Dihydrogenphosphat, Chlorid und Tetrafluoroborat.

Insgesamt haben sich Phasentransferkatalysatoren als besonders günstig herausgestellt, die eine relative hohe Hydrophilie aufweisen. Dies wird damit erklärt, dass die polare wässrige Reduktionsmittel enthaltende Phase an der Grenzfläche zum auf der natürlichen Zahnsubstanz aufgetragenen Adhäsiv im Vergleich zur hydrophoben Dentalmaterial-Masse relativ klein ist. Mit anderen Worten ist nur ein sehr geringer Anteil des Phasentransferkatalysators in der wässrigen Phase vorhanden. Entsprechend führt eine hohe Hydrophilie dazu, dass sich auch ein erheblicher Anteil in der wässrigen Phase befindet und beschleunigt damit das Einbringen des Reduktionsmittels in die hydrophobe Monomerenmasse. Entsprechend ist es bevorzugt, polare Substanzen als Phasentransferkatalysatoren einzusetzen, wobei die Kationen und Anionen vorzugsweise nach dem HSAB-Konzept ("Hard and Soft Acids and Bases") als eher hart charakterisiert werden. Solche harten Kationen sind vorzugsweise Ammoniumionen, mit relativ kurzkettigen (C₁ bis C₄) Resten, wobei einzelne längere Reste (bis C₂₀) sich als unproblematisch herausgestellt haben. Gleiches gilt für die verwendeten Anionen, die vorzugsweise hart sind, bspw. Tetrafluorborate, während weiche Anionen, bspw. Hexafluorophosphate, sich als weniger geeignet herausgestellt haben.

In einer ganz besonders bevorzugten Ausführungsform umfasst der Phasentransferkatalysator Tetrabutylammoniumhydrogensulfat, Tetrahexylammoniumhydrogensulfat, Tetramethylammoniumhydrogensulfat, Tetraphenylphosphoniumchlorid, Tetrabutylphosphoniumchlorid, Bis-[tetrakis(hydroxymethyl)phosphonium]sulfat, 1,2,3-Trimethylimidazoliummethylsulfat, 1,2,3-Trimethylimidazoliumethylsulfat, 1-Ethyl,2, 3-dimethyl-imidazoliumethylsulfat, Cetyltrimethylammoniumhydrogensulfat, (Vinylbenzyl)trimethylammoniumchlorid, Tetrabutylammoniumchlorid, Tetrabutylammoniumacetat, Tetrabutylammoniumhexafluorophosphatund/oder Tetrabutylammoniumtetrafluoroborat,.

Als Füllstoffe für die Katalysator- und die Basispaste können anorganische oder organische Materialien verwendet werden. Bei den Füllstoffen kann es sich um verstärkende Füllstoffe oder um nicht-verstärkende Füllstoffe oder um Gemische davon handeln.

Als verstärkende Füllstoffe eignen sich insbesondere hochdisperse, aktive Füllstoffe mit einer BET-Oberfläche von wenigstens 50 m²/g. Besonders geeignet sind solche mit einer Einzelpartikelgröße im Nanometerbereich, welche als Aggregate und/oder Agglomerate vorliegen können. Bevorzugte verstärkende Füllstoffe sind Substanzen, die ausgewählt sind aus der Gruppe bestehend aus Aluminiumhydroxid, Zinkoxid, Titandioxid, Zirkoniumoxid, Siliciumdioxid sowie gefällter und/oder pyrogener Kieselsäure. Selbstverständlich können die zuvor genannten Verbindungen einzeln oder in beliebiger Kombination miteinander eingesetzt werden, und zwar auch sowohl in hydrophiler als auch in hydrophobierter Form.

Ferner bevorzugt liegt der wenigstens eine verstärkende Füllstoff in Form von Nanopartikeln, als faser- oder blättchenförmiger Füllstoff, beispielsweise als mineralischer, faserförmiger Füllstoff, oder als synthetischer, faserförmiger Füllstoff vor.

Der Anteil an verstärkendem Füllstoff an dem erfindungsgemäßen Dentalmaterial beträgt üblicherweise 0,1 bis 50 Gew.-%, bevorzugt 0,2 bis 20 Gew.-% und besonders bevorzugt 0,5 bis 10 Gew.-%, bezogen auf das gesamte Dentalmaterial.

Als nichtverstärkende Füllstoffe eignen sich prinzipiell dieselben Substanzen wie für die verstärkende Füllstoffe, wobei die nichtverstärkenden jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugte nichtverstärkende Füllstoffe sind Substanzen, die ausgewählt werden aus der Gruppe bestehend aus Dentalgläsern, die vorzugsweise röntgenopake Inhaltsstoffe enthalten, Erdalkalimetalloxiden, Erdalkalimetallhydroxiden, Erdalkalimetallfluoriden, Erdalkalimetallcarbonaten, Calciumapatit (Ca₅[(F, Cl, OH, ½CO₃)|(PO₄)₃], insbesondere Calciumhydroxylapatit (Ca₅[(OH)|(PO₄)₃], Titandioxid, Zirkoniumoxid, Aluminiumhydroxid, Siliciumdioxid (z.B. Cristobalit, Quarzgut) gefällte Kieselsäure und Calciumcarbonat. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nichtverstärkenden Füllstoffe eine mittlere Korngröße von größer als 0,1 µm (Ullmann Encyclopädie der Technischen Chemie, Band 21, Seite 523) auf.

Der Anteil an nichtverstärkendem Füllstoff im erfindungsgemäßen Dentalmaterial beträgt üblicherweise 5 bis 80 Gew.-%, bevorzugt 10 bis 70 Gew.-% und besonders bevorzugt 20 bis 70 Gew.-%, bezogen auf das gesamte Dentalmaterial.

Weiterhin können auch größere Mengen an ausgewählten röntgenopaken Füllstoffen in der wenigstens einen Basispaste und/oder der wenigstens einen Katalysatorpaste vorhanden sein. Dabei handelt es sich vorzugsweise um unregelmäßig geformtes oder sphärisches YbF₃- oder YF₃-Pulver mit einer mittleren Korngröße der Primärpartikel von 40 nm bis 1,5 µm und besonders bevorzugt um Kern-Schale-Kombinationsprodukte aus YF₃- oder YbF₃-Kern und SiO₂-Schale, wobei ganz besonders bevorzugt die SiO₂₋Schalenoberfläche silanisiert ist. Insbesondere weist ein solches Kern-Schale-Kombinationsprodukt einen Brechungsindex von 1,48 bis 1,54, eine mit einem Laserbeugungspartikelgrößenmessgerät SALD-2001 (Schimadzu) gemessene mittlere Korngröße der agglomerierten Partikel zwischen 0,5 und 5 µm und eine B.E.T. Oberfläche, gemessen mit einem Tristar 3000 Gerät von Micromeritics von 2 bis 5 m²/g auf. Dabei liegt der Brechungsindex des Kern-Schale-Kombinationsproduktes aus YbF₃-Kern und SiO₂₋Schale zwischen 1,52 und 1,54.

Der Gesamtanteil an verstärkenden und nichtverstärkenden Füllstoffen im erfindungsgemäßen Dentalmaterial beträgt üblicherweise 30 bis 80 Gew.-%, bevorzugt 40 bis 80 Gew.-%, besonders bevorzugt 50 bis 75 Gew.-%, bezogen auf das gesamte Dentalmaterial.

Vorzugsweise enthält die wenigstens eine Basiskomponente und/oder die wenigstens eine Katalysatorkomponente einen oder mehrere Zusatzstoffe, wie z.B. Puffersalze, Wasserfänger, Metallfänger, Metallkomplexbildner, weitere Pastenbildner, Tenside, Wirkstoffe, Diagnostik ermöglichende Substanzen, Zahnsubstanz ätzende und/oder adhäsiv wirkende Substanzen wie z.B. MDP (Methacryloyldecylphosphat), Fluoridisierungsmittel, Desensibilisierungsmittel, Haftverbundvermittler, Farbstoffe, Farbpigmente, Fluoreszenzfarbstoffe, weitere Initiatoren oder Initiatorkomponenten, Stabilisatoren, Polymerisationsinhibitoren, Thixotropiehilfsmittel sowie antibakterielle Substanzen.

Der Gewichtsanteil der Zusatzstoffe bezogen auf die Gesamtmasse der wenigstens einen Basiskomponente und/oder der wenigstens einen Katalysatorkomponente beträgt in der Regel 0 bis 20 Gew.-%, bezogen auf die Gesamtmasse der jeweiligen Komponente, vorzugsweise 0,0001 bis 15 Gew.-% und besonders bevorzugt von 0,001 bis 10 Gew.-%.

Das erfindungsgemäße Mehrkomponentensystem wird vorzugsweise in geeigneten Primärverpackungen, wie in Kartuschen, Kompulen und 2-Komponenten-Spritzen, wie sie z.B. in der EP 2,190,592 B2 beschrieben sind, gelagert und ist auf die spätere Verwendung zugeschnitten proportioniert.

In einer besonders bevorzugten Ausführungsform weist das erfindungsgemäße Mehrkomponentensystem enthaltend eine in einer Primärverpackung abgefüllte Katalysatorpaste (A) und eine in einer Primärverpackung abgefüllte Basispaste (B) bei einer Lagerung bei 23°C eine Lagerstabilität von mindestens 15 Monaten auf.

Lagerstabilität bedeutet in diesem Zusammenhang, dass die Haftfestigkeit des gehärteten Dentalmaterials in Verbindung mit einem Dentaladhäsiv an Dentin, zu dessen Herstellung ein gelagertes Mehrkomponentensystem verwendet wurde, sich nicht signifikant von der Haftfestigkeit des gehärteten Dentalmaterials in Verbindung mit demselben Dentaladhäsiv an Dentin unterscheidet, zu dessen Herstellung dasselbe Mehrkomponentensystem zu Beginn der Lagerung verwendet wurde. Weiterhin bedeutet Lagerstabilität eine für die Leistungsfähigkeit des Dentalmaterials nur unbedeutende Änderung der Abbindekinetik, der rheologischen Eigenschaften und der mechanischen Eigenschaften gegenüber den Eigenschaften zu Beginn der Lagerung.

Die erfindungsgemäße Dentalmasse kommt häufig zusammen mit einem Dentaladhäsiv zum Einsatz, insbesondere in Kombination mit einem Prime + Bond oder All-in-one-Adhäsiv.

Die Erfindung betrifft daher auch ein Baukastensystem (kit-of-parts) umfassend oben beschriebenes polymerisierbares Dentalmaterial und ein Dentaladhäsiv.

Die Erfindung betrifft auch ein gehärtetes Dentalmaterial, welches durch Vermischen der erfindungsgemäßen Katalysatorpaste (A) und Basispaste (B) vorzugsweise im Verhältnis 1:20 bis 1:1 und durch Polymerisation des polymerisierbaren Dentalmaterials erhalten wird.

Die Erfindung betrifft auch die Verwendung eines polymerisierbaren Dentalmaterials enthaltend eine oben beschriebene Katalysatorpaste (A) und eine oben beschriebene Basispaste (B) zur Herstellung eines Stumpfaufbaumaterials, eines polymerisierbaren Kompositzements und/oder eines Bulk-Fill Komposits für die Herstellung von Stumpfaufbauten, Befestigungen und/oder Zahnfüllungen.

Die Erfindung betrifft ferner die Verwendung eines polymerisierbaren Dentalmaterials enthaltend eine oben beschriebene Katalysatorpaste (A) und eine oben beschriebene Basispaste (B) als Stumpfaufbaumaterial, als polymerisierbarer Kompositzement und/oder als Bulk-Fill Komposit.

Weiterbildungen, Vorteile und Anwendungsmöglichkeiten der Erfindung ergeben sich auch aus der nachfolgenden Beschreibung und der Ausführungsbeispiele. Dabei bilden alle beschriebenen Merkmale für sich oder in beliebiger Kombination den Gegenstand der Erfindung unabhängig von ihrer Zusammenfassung in den Ansprüchen oder deren Rückbeziehung.

### Ausführungsbeispiele

Zur Herstellung der 1-Schritt-1-Komponenten lichthärtenden, selbstätzenden und selbstadhäsiven Bondings werden die in den nachstehenden Tabellen 1 und 2 aufgeführten Inhaltsstoffe verwendet. Alle Inhaltsstoffe außer Wasser und Ethanol wurden in einen Becher eingewogen und mit einem Zentrifugalmischer (Hauschild DAC 150 FVZ) homogenisiert. Anschließend werden diese mit einem Dreiwalzwerk Exakt 80E dispergiert. Nach der Dispergierung werden Wasser und Ethanol zugegeben, das Material erneut mit dem Zentrifugalmischer homogenisiert und in schwarze 10 ml Tropfflaschen (Fa. Transcodent) umgefüllt.

Zur Herstellung der dualhärtenden Stumpfaufbaukomposite und Kompositzemente wurden die in den nachstehenden Tabellen 3 bis 21 aufgeführten Inhaltsstoffe verwendet.

Alle Inhaltsstoffe werden in einen Becher eingewogen und mit einem Zentrifugalmischer (Hauschild DAC 150 FVZ) homogenisiert. Anschließend erfolgt die Dispergierung auf einem Dreiwalzwerk (Exakt 80E) bei Raumtemperatur. Anschließend werden die Pasten erneut in dem Zentrifugalmischer homogenisiert.

**Tabelle 1: 1-Schritt-1-Komponenten lichthärtendes Bonding ohne DHEPT - entspricht Bonding I (Herstellungsbeispiel I); angelehnt an EP 2 554 154 A1, Comparative Example 2**

| **Inhaltsstoff** | **Menge [Gew.-%]** |
|---|---|
| BisGMA⁷⁾ | 28,85 |
| HEMA⁸⁾ | 22,50 |
| GDMA⁹⁾ | 4,50 |
| CQ²⁾ | 1,80 |
| EPD¹⁾ | 0,90 |
| MDP¹⁴⁾ | 9,00 |
| BHT³⁾ | 0,05 |
| HDK H2000 | 4,50 |
| Wasser | 13,50 |
| Ethanol | 13,50 |
| BAPO¹³⁾ | 0,90 |
| DHEPT¹¹⁾ | 0,00 |
| Summe: | 100,00 |

**Tabelle 2: 1-Schritt-1-Komponenten lichthärtendes Bonding mit DHEPT - entspricht Bonding II (Herstellungsbeispiel II); angelehnt an EP 2 554 154 A1, Example 5**

| **Inhaltsstoff** | **Menge [Gew.-%]** |
|---|---|
| BisGMA⁷⁾ | 27,05 |
| HEMA⁸⁾ | 22,50 |
| GDMA⁹⁾ | 4,50 |
| CQ²⁾ | 1,80 |
| EPD¹⁾ | 0,90 |
| MDP¹⁴⁾ | 9,00 |
| BHT³⁾ | 0,05 |
| HDK H2000 | 4,50 |
| Wasser | 13,50 |
| Ethanol | 13,50 |
| BAPO¹³⁾ | 0,90 |
| DHEPT¹¹⁾ | 1,80 |
| Summe: | 100,00 |

**Tabelle 3: Polymerisierbares Dentalmaterial Katalysatorpaste (Herstellungsbeispiel 1)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 4,50 |
| YbF₃(100 nm) | 20,00 |
| Cristobalitmehl (6 µm mit Methacrylsilan) | 41,61 |
| BPO¹⁰⁾ | 0,80 |
| BHT³⁾ | 0,09 |
| Summe: | 100,00 |

**Tabelle 4: Polymerisierbares Dentalmaterial Basispaste (Herstellungsbeispiel 2)**

| **Inhaltsstoff** | **Menge [Gew.-%]** |
|---|---|
| UDMA⁴⁾ | 24,75 |
| DDDDMA⁵⁾ | 6,25 |
| TM PTMA⁶⁾ | 2,00 |
| HDK H2000 | 4,50 |
| YbF₃ (100 nm) | 20,00 |
| Cristobalitmehl (6 µm mit Methacrylsilan) | 41,10 |
| DHEPT¹¹⁾ | 0,90 |
| EPD¹⁾ | 0,20 |
| CQ²⁾ | 0,09 |
| BHT³⁾ | 0,01 |
| HMBP¹²⁾ | 0,20 |
| Summe: | 100,00 |

**Tabelle 5: Polymerisierbares Dentalmaterial Basispaste (Herstellungsbeispiel 3)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 4,50 |
| YbF₃ (100 nm) | 20,00 |
| Cristobalitmehl (6 µm mit Methacrylsilan) | 39,10 |
| DHEPT¹¹⁾ | 0,90 |
| EPD¹⁾ | 0,20 |
| CQ²⁾ | 0,09 |
| Natriumsulfit (5 µm) | 2,00 |
| BHT³⁾ | 0,01 |
| HMBP¹²⁾ | 0,20 |
| Summe: | 100,00 |

**Tabelle 6: Polymerisierbares Dentalmaterial Katalysatorpaste (Herstellungsbeispiel 4)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,93 |
| BPO¹⁰⁾ | 0,80 |
| BHT³⁾ | 0,09 |
| Tetraethylammonium p-toluolsulfonat (nicht erfindungsgemäßer Phasentransferkatalysator) | 0,18 |
| Summe: | 100,00 |

**Tabelle 7: Polymerisierbares Dentalmaterial Katalysatorpaste (Herstellungsbeispiel 5)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,91 |
| BPO¹⁰⁾ | 0,80 |
| BHT³⁾ | 0,09 |
| Tetrabutylammoniumchlorid (erfindungsgemäßer Phasentransferkatalysator) | 0,20 |
| Summe: | 100,00 |

**Tabelle 8: Polymerisierbares Dentalmaterial Katalysatorpaste (Herstellungsbeispiel 6)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,91 |
| BPO¹⁰⁾ | 0,80 |
| BHT³⁾ | 0,09 |
| (Vinylbenzyl)trimethylammoniumchlorid (erfindungsgemäßer Phasentransferkatalysator) | 0,20 |
| Summe: | 100,00 |

**Tabelle 9: Polymerisierbares Dentalmaterial Katalysatorpaste (Herstellungsbeispiel 7)**

| **Inhaltsstoff** | **Menge [Gew.-%]** |
|---|---|
| UDMA⁴⁾ | 24,75 |
| DDDDMA⁵⁾ | 6,25 |
| TM PTMA⁶⁾ | 2,00 |
| HDK H2000 | 2,00 |
| YbF₃ (100 nm) | 20,00 |
| Cristobalitmehl (6 µm mit Methacrylsilan) | 43,91 |
| BPO¹⁰⁾ | 0,80 |
| BHT³⁾ | 0,09 |
| Tetrabutylammoniumhydrogensulfat (erfindungsgemäßer Phasentransferkatalysator) | 0,20 |
| Summe: | 100,00 |

**Tabelle 10: Polymerisierbares Dentalmaterial Katalysatorpaste (Herstellungsbeispiel 8)**

| **Inhaltsstoff** | **Menge [Gew.-%]** |
|---|---|
| UDMA⁴⁾ | 24,75 |
| DDDDMA ⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 44,01 |
| BPO¹⁰⁾ | 0,80 |
| BHT³⁾ | 0,09 |
| Tetrabutylammoniumhydrogensulfat (erfindungsgemäßer Phasentransferkatalysator) | 0,10 |
| Summe: | 100,00 |

**Tabelle 11: Polymerisierbares Dentalmaterial Katalysatorpaste (Herstellungsbeispiel 9)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,71 |
| BPO¹⁰⁾ | 0,80 |
| BHT³⁾ | 0,09 |
| Tetrabutylammoniumhydrogensulfat (erfindungs-gemäßer Phasentransferkatalysator) | 0,40 |
| Summe: | 100,00 |

**Tabelle 12: Polymerisierbares Dentalmaterial Katalysatorpaste (Herstellungsbeispiel 10)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 44,01 |
| BPO¹⁰⁾ | 0,80 |
| BHT³⁾ | 0,09 |
| Tetramethylammoniumhydrogensulfat (erfindungsgemäßer Phasentransferkatalysator) | 0,10 |
| Summe: | 100,00 |

**Tabelle 13: Polymerisierbares Dentalmaterial Katalysatorpaste (Herstellungsbeispiel 11)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,84 |
| BPO¹⁰⁾ | 0,80 |
| BHT³⁾ | 0,09 |
| Tetrahexylammoniumhydrogensulfat (erfindungsgemäßer Phasentransferkatalysator) | 0,27 |
| Summe: | 100,00 |

**Tabelle 14: Polymerisierbares Dentalmaterial Katalysatorpaste (Herstellungsbeispiel 12)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,88 |
| BPO¹⁰⁾ | 0,80 |
| BHT³⁾ | 0,09 |
| Cetyltrimethylammoniumhydrogensulfat (erfindungsgemäßer Phasentransferkatalysator) | 0,23 |
| Summe: | 100,00 |

**Tabelle 15: Polymerisierbares Dentalmaterial Katalysatorpaste (Herstellungsbeispiel 13)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,93 |
| BPO¹⁰⁾ | 0,80 |
| BHT³⁾ | 0,09 |
| Tetrabutylammoniumacetat (erfindungsgemäßer Phasentransferkatalysator) | 0,18 |
| Summe: | 100,00 |

**Tabelle 16: Polymerisierbares Dentalmaterial Katalysatorpaste (Herstellungsbeispiel 14)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,91 |
| BPO¹⁰⁾ | 0,80 |
| BHT³⁾ | 0,09 |
| Tetrabutylammoniumtetrafluoroborat (erfindungsgemäßer Phasentransferkatalysator) | 0,20 |
| Summe: | 100,00 |

**Tabelle 17: Polymerisierbares Dentalmaterial Katalysatorpaste (Herstellungsbeispiel 15)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,88 |
| BPO¹⁰⁾ | 0,80 |
| BHT³⁾ | 0,09 |
| Tetrabutylammoniumhexafluorophosphat (erfindungsgemäßer Phasentransfer-katalysator) | 0,23 |
| Summe: | 100,00 |

**Tabelle 18: Polymerisierbares Dentalmaterial Katalysatorpaste (Herstellungsbeispiel 16)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,91 |
| BPO¹⁰⁾ | 0,80 |
| BHT³⁾ | 0,09 |
| 1-Ethyl-2,3-dimethyl-imidazolium-ethylsulfat (erfindungsgemäßer Phasentransferkatalysator) | 0,20 |
| Summe: | 100,00 |

**Tabelle 19: Polymerisierbares Dentalmaterial Katalysatorpaste (Herstellungsbeispiel 17)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,87 |
| BPO¹⁰⁾ | 0,80 |
| BHT³⁾ | 0,09 |
| Bis[tetrakis(hydroxymethyl)phosphonium]sulfat (erfindungsgemäßer Phasentransfer-katalysator) | 0,24 |
| Summe: | 100,00 |

**Tabelle 20: Polymerisierbares Dentalmaterial Katalysatorpaste (Herstellungsbeispiel 18)**

| **Inhaltsstoff** | **Menge [Gew.-%]** |
|---|---|
| UDMA⁴⁾ | 24,75 |
| DDDDMA⁵⁾ | 6,25 |
| TMPTMA⁶⁾ | 2,00 |
| HDK H2000 | 2,00 |
| YbF₃ (100 nm) | 20,00 |
| Cristobalitmehl (6 µm mit Methacrylsilan) | 43,89 |
| BPO¹⁰⁾ | 0,80 |
| BHT³⁾ | 0,09 |
| Tetraphenylphosphoniumchlorid (erfindungsgemäßer Phasentransferkatalysator) | 0,22 |
| Summe: | 100,00 |

**Tabelle 21: Polymerisierbares Dentalmaterial Katalysatorpaste (Herstellungsbeispiel 19)**

| **Inhaltsstoff** | **Menge [Gew.-%]** |
|---|---|
| UDMA⁴⁾ | 24,75 |
| DDDDMA⁵⁾ | 6,25 |
| TM PTMA⁶⁾ | 2,00 |
| HDK H2000 | 2,00 |
| YbF₃ (100 nm) | 20,00 |
| Cristobalitmehl (6 µm mit Methacrylsilan) | 43,88 |
| BPO¹⁰⁾ | 0,80 |
| BHT³⁾ | 0,09 |
| (4-Methylthiophenyl)methyl phenyl sulfonium triflat (erfindungsgemäßer Phasentransferkatalysator) | 0,23 |
| Summe: | 100,00 |

| | |
|---|---|
| ¹⁾ Bei EPD handelt es sich um Ethyl-4-dimethylaminobenzoat. ²⁾ Bei CQ handelt es sich um D,L-Campherchinon. ³⁾ Bei BHT handelt es sich um 2,6-Di-tert-butyl-4-methylphenol. ⁴⁾ Bei UDMA handelt es sich um ein Isomerengemisch aus Di-2-methacryloxyethyl-2,2,4-trimethylhexamethylendicarbamat und Di-2-(meth)acryl-oxyethyl-2,4,4-trimethyl-hexa-methylendicarbamat entsprechend Formel (I). 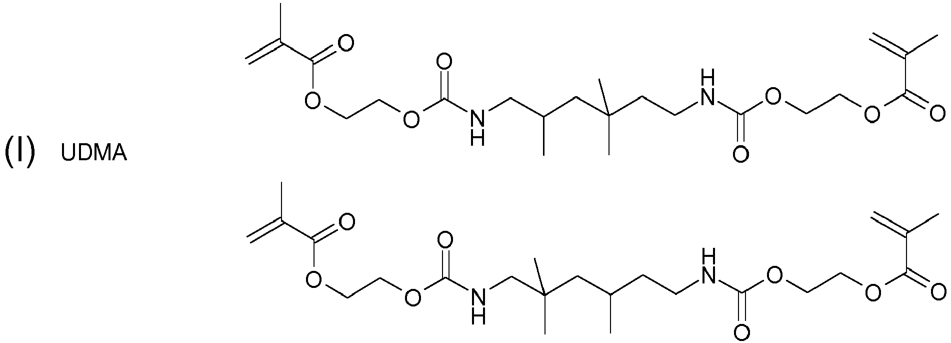 ⁵⁾ Bei DDDDMA handelt es sich um 1,12-Dodecandioldimethacrylat. ⁶⁾ Bei TMPTMA handelt es sich um Trimethylolpropantrimethacrylat. ⁷⁾ Bei BisGMA handelt es sich um Bisphenol-A-Glycidylmetharcrylat. ⁸⁾ Bei HEMA handelt es sich um 2-Hydoxyethylmethacrylat. ⁹⁾ Bei GDMA handelt es sich um Glycerin-1,3-dimethacrylat. ¹⁰⁾ Bei BPO handelt es sich um Dibenzoylperoxid. ¹¹⁾ Bei DHEPT handelt es sich um p-Tolyldiethanolamine. ¹²⁾ Bei HMBP handelt es sich um 2-Hydroxy-4-methoxy-benzophenon. ¹³⁾ Bei BAPO handelt es sich um Bis(2,4,6-trimethylbenzoyl)phenylphosphinoxid, das als Hilfsinitiator für die Lichthärtung fungiert. ¹⁴⁾ Bei MDP handelt es sich um 10-Methacryloyl-oxdecyl-dihydrogenphosphat. | |

Zur Herstellung der Patent- und Vergleichsbeispiele wurden die Katalysator- und Basispastenformulierungen entsprechend den Kombinationen von Tabelle 19 in 5 ml Doppelspritzen Typ Mixpac 1:1 SDL X05-01-52 (Sulzer) abgefüllt. Zur Prüfung werden die Dentalmassen über einen statischen Mischer Typ Mixpac MLT 2.5-10-D (Sulzer) mit Endo Tip Typ IOR 209-20 ausgetragen.

**Tabelle 22: Kombination der Herstellungsbeispiele**

| **Katalysatorpaste** | **Basispaste** | **Kombination** |
|---|---|---|
| Herstellungsbeispiel 1 | Herstellungsbeispiel 2 | Vergleichsbeispiel 1 |
| Herstellungsbeispiel 1 | Herstellungsbeispiel 3 | Vergleichsbeispiel 2 |
| Herstellungsbeispiel 4 | Herstellungsbeispiel 3 | Vergleichsbeispiel 3 |
| Herstellungsbeispiel 5 | Herstellungsbeispiel 3 | Patentbeispiel 1 |
| Herstellungsbeispiel 6 | Herstellungsbeispiel 3 | Patentbeispiel 2 |
| Herstellungsbeispiel 7 | Herstellungsbeispiel 3 | Patentbeispiel 3 |
| Herstellungsbeispiel 8 | Herstellungsbeispiel 3 | Patentbeispiel 4 |
| Herstellungsbeispiel 9 | Herstellungsbeispiel 3 | Patentbeispiel 5 |
| Herstellungsbeispiel 10 | Herstellungsbeispiel 3 | Patentbeispiel 6 |
| Herstellungsbeispiel 11 | Herstellungsbeispiel 3 | Patentbeispiel 7 |
| Herstellungsbeispiel 12 | Herstellungsbeispiel 3 | Patentbeispiel 8 |
| Herstellungsbeispiel 13 | Herstellungsbeispiel 3 | Patentbeispiel 9 |
| Herstellungsbeispiel 14 | Herstellungsbeispiel 3 | Patentbeispiel 10 |
| Herstellungsbeispiel 15 | Herstellungsbeispiel 3 | Patentbeispiel 11 |
| Herstellungsbeispiel 16 | Herstellungsbeispiel 3 | Patentbeispiel 12 |
| Herstellungsbeispiel 17 | Herstellungsbeispiel 3 | Patentbeispiel 13 |
| Herstellungsbeispiel 18 | Herstellungsbeispiel 3 | Patentbeispiel 14 |
| Herstellungsbeispiel 19 | Herstellungsbeispiel 3 | Patentbeispiel 15 |

Zur Prüfung des Haftverbundes wurden die Stumpfaufbaumaterialien aus den Vergleichsbeispielen 1 bis 3 und den Patentbeispielen 1 bis 15, und das Vergleichsmaterial LuxaCore Smartmix Dual (DMG, LOT 707376) mit den Bondings I und II kombiniert. Die Versuche erfolgten an menschlichem Dentin in Anlehnung an ISO 29022.

Dazu wurden menschliche Zähne in Kalteinbettmittel (VariKwick, Fa. Bühler) eingebettet und das Dentin durch Beschleifen der Oberfläche freigelegt. Auf die so behandelten Prüfkörper wurde das Bonding aufgetragen und 10s lichtgehärtet (Superlite 1100, Fa. M+W Dental). Anschließend wurden die Prüfkörper in eine Verbundschraubzwinge (Bonding Clamp, Fa. Ultradent) eingefügt, die eine Einsteckform für den anzubringenden Stumpfaufbauzylinder (Bonding Mold Insert, Fa. Ultradent) enthält. Die Füllmulde der Form wurde über einer für den Verbund geeigneten Stelle auf dem Zahn mittig angeordnet und abgesenkt. Als nächstes wurde das Stumpfaufbaumaterial auf die Verbundfläche aufgebracht und im Selbsthärtungsmodus für 7 min im Dunklen bei 37°C gelagert. Nach dieser Zeit wurden die Prüfkörper aus der Form entnommen und für weitere 24 h bei 37°C in Wasser gelagert.

Nach Ablauf dieser Zeit erfolgte die Abscherungsprüfung an einer Universalprüfmaschine (Zwicki Universalprüfmaschine Z0.5 TN) mit entsprechendem Aufbau. Dazu wurden die Verbundprüfkörper in eine Probehalterung (Test Base Clamp, Fa. Ultradent) eingespannt. Die Prüfkörper wurden in der Halterung unter ein Querhaupt mit ausgesparter Klinge (Crosshead Assembly, Fa. Ultradent) mittig am Kompositzylinder und am Zahn anliegend ausgerichtet. Die Prüfkörper wurden mit einer Prüfgeschwindigkeit von 1,0 mm/min bis zum Bruch belastet. Die Ergebnisse der Prüfungen sind in Tabelle 23 dargestellt.

**Tabelle 23 : Ergebnisse**

| Polymerisierbares Dentalmaterial | Adhäsiv | Haftverbund |
|---|---|---|
| | | an Dentin* |
| | | [MPa] |
| | | |
| Nicht erfindungsgemäße Beispiele: | | |
| LuxaCore Smartmix Dual LOT 707376 weiß | Herstellbeispiel I: Bonding I | 7,6 |
| LuxaCore Smartmix Dual LOT 707376 weiß | Herstellbeispiel II: Bonding II | 3,9 |
| Vergleichsbeispiel 1: polymerisierbares Dentalmaterial | Herstellbeispiel I: Bonding I | 7,3 |
| Vergleichsbeispiel 1: polymerisierbares Dentalmaterial | Herstellbeispiel II: Bonding II | 3,8 |
| Vergleichsbeispiel 2: polymerisierbares Dentalmaterial -enthält Natriumsulfit (15,87 mmol) | Herstellbeispiel I: Bonding I | 8,3 |
| Vergleichsbeispiel 2: polymerisierbares Dentalmaterial -enthält Natriumsulfit (15,87 mmol) | Herstellbeispiel II: Bonding II | 9,5 |
| Vergleichsbeispiel 3: polymerisierbares Dentalmaterial -enthält Natriumsulfit (15,87 mmol) und Tetraethylammonium p-toluolsulfonat (0,59 mmol; Phasentransferkatalysator) | Herstellbeispiel I: Bonding I | 8,6 |
| | | |
| Erfindungsgemäße Beispiele mit Ammoniumsalzen als Phasentransferkatalysatoren: | | |
| Patentbeispiel 1: polymerisierbares Dentalmaterial - enthält Natriumsulfit (15,87 mmol) und Tetrabutylammoniumchlorid (0,72 mmol; Phasentransferkatalysator) | Herstellbeispiel I: Bonding I | 18,3 |
| Patentbeispiel 2: polymerisierbares Dentalmaterial - enthält Natriumsulfit (15,87 mmol) und (Vinylbenzyl)trimethylammoniumchlorid (0,94 mmol; Phasentransferkatalysator) | Herstellbeispiel I: Bonding I | 12,8 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | Herstellbeispiel I: Bonding I | 15,3 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) nach 15 Monaten Lagerung bei 23 ± 1 °C | Herstellbeispiel I: Bonding I | 15,7 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | Herstellbeispiel II: Bonding II | 14,6 |
| Patentbeispiel 4: polymerisierbares Dentalmaterial - enthält Natriumsulfit (15,87 mmol) und Tetrabutylammoniumhydrogensulfat (0,30 mmol; Phasentransferkatalysator) | Herstellbeispiel I: Bonding I | 13,3 |
| Patentbeispiel 5: polymerisierbares Dentalmaterial - enthält Natriumsulfit (15,87 mmol) und Tetrabutylammoniumhydrogensulfat (1,18 mmol; Phasentransferkatalysator) | Herstellbeispiel I: Bonding I | 15,8 |
| Patentbeispiel 6: polymerisierbares Dentalmaterial - enthält Natriumsulfit (15,87 mmol) und Tetramethylammoniumhydrogensulfat (0,59 mmol; Phasentransferkatalysator) | Herstellbeispiel I: Bonding I | 14,5 |
| Patentbeispiel 7: polymerisierbares Dentalmaterial - enthält Natriumsulfit (15,87 mmol) und Tetrahexylammoniumhydrogensulfat (0,59 mmol; Phasentransferkatalysator) | Herstellbeispiel I: Bonding I | 13,4 |
| Patentbeispiel 8: polymerisierbares Dentalmaterial - enthält Natriumsulfit (15,87 mmol) und Cetyltrimethylammoniumhydrogensulfat (0,59 mmol; Phasentransferkatalysator) | Herstellbeispiel I: Bonding I | 12,8 |
| Patentbeispiel 9: polymerisierbares Dentalmaterial - enthält Natriumsulfit (15,87 mmol) und Tetrabutylammoniumacetat (0,59 mmol; Phasentransferkatalysator) | Herstellbeispiel I: Bonding I | 13,0 |
| Patentbeispiel 10: polymerisierbares Dentalmaterial - enthält Natriumsulfit (15,87 mmol) und Tetrabutylammoniumtetrafluoroborat (0,61 mmol; Phasentransferkatalysator) | Herstellbeispiel I: Bonding I | 14,4 |
| Patentbeispiel 11: polymerisierbares Dentalmaterial - enthält Natriumsulfit (15,87 mmol) und Tetrabutylammoniumhexafluorophosphat (0,59 mmol; Phasentransferkatalysator) | Herstellbeispiel I: Bonding I | 10,5 |
| | | |
| Erfindungsgemäße Beispiele mit heterozyklischem Ammoniumsalz als Phasentransferkatalysator: | | |
| Patentbeispiel 12: polymerisierbares Dentalmaterial - enthält Natriumsulfit (15,87 mmol) und 1-Ethyl-2,3-dimethyl-imidazoliumethylsulfat (0,8 mmol; Phasentransferkatalysator) | Herstellbeispiel I: Bonding I | 11,2 |
| | | |
| Erfindungsgemäße Beispiele mit Phosphoniumsalzen als Phasentransferkatalysatoren: | | |
| Patentbeispiel 13: polymerisierbares Dentalmaterial - enthält Natriumsulfit (15,87 mmol) und Bis[tetrakis(hydroxymethyl)phosphonium]sulfat (0,59 mmol; Phasentransferkatalysator) | Herstellbeispiel I: Bonding I | 11,7 |
| Patentbeispiel 14: polymerisierbares Dentalmaterial - enthält Natriumsulfit (15,87 mmol) und Tetraphenylphosphoniumchlorid (0,59 mmol; Phasentransferkatalysator) | Herstellbeispiel I: Bonding I | 10,5 |
| | | |
| Erfindungsgemäße Beispiele mit Sulfoniumsalz als Phasentransferkatalysator: | | |
| Patentbeispiel 15: polymerisierbares Dentalmaterial - enthält Natriumsulfit (15,87 mmol) und (4-Methylthiophenyl)methyl phenyl sulfonium triflat (0,59 mmol; Phasentransferkatalysator) | Herstellbeispiel I: Bonding I | 14,0 |
| | | |
| Erfindungsgemäßes Beispiel mit verschiedenen kommerziellen All-in-One Adhäsiven: | | |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | Clearfil S3 Bond Plus (Kuraray) | 19,5 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | Clearfil Universal Bond (Kuraray) | 14,9 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | Scotchbond Universal (3M Espe) | 16,2 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | Xeno Select (Dentsply) | 11,6 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | All Bond Universal (Bisco) | 11,0 |
| Patentbeispiel 3: polymerisierbares Dentalmaterial - enthält Natriumsulfit (15,87 mmol) und Tetrabutylammoniumhydrogensulfat (0,59 mmol; Phasentransferkatalysator) | iBond Universal (Heraeus Kulzer) | 10,1 |

| | | |
|---|---|---|
| *Anzahl nicht haftender Prüfkörper wurden als Nullwert eingerechnet | | |

Aus Tabelle 23 sind folgende Erkenntnisse zu entnehmen:
- Das kommerziell erhältliche und in Deutschland marktführende Stumpfaufbaumaterial "LuxaCore Smartmix Dual" zeigt mit beiden Bonding-Varianten (Bonding I, gemäß EP 2 554 154 A1, Comparative Example 2, Seite 19; Bonding II, gemäß EP 2 554 154 A1, Example 5, Seite 31) einen niedrigen Haftverbund. Der Haftverbund mit Bonding II ist hierbei nochmals deutlich gegenüber Bonding I erniedrigt, was damit erklärt werden kann, dass bei Bonding II ein Teil der ätzenden Wirkung des Phosphorsäuremethacrylates durch das zusätzlich enthaltene aromatische Amin DHEPT neutralisiert wird.
- Das in Vergleichsbeispiel 1 beschriebene polymerisierbare Dentalmaterial, (Initiator-Zusammensetzung ohne das wasserlösliche Reduktionsmittel vergleichbar mit Comparative Example 3, Seite 28, aus EP 2 554 154 A1) zeigt völlig analog zu "Luxacore Smartmix Dual" mit beiden Bonding-Varianten einen niedrigen Haftverbund. Auch hier ist der Haftverbund mit Bonding II nochmals deutlich gegenüber Bonding I erniedrigt. Dies zeigt, dass die in allen Beispielen verwendete Zusammensetzung hinsichtlich der Monomeren und Füllstoffe in Bezug auf die Haftkraft mit anderen kommerziellen Stumpfaufbaumaterialien vergleichbar ist.
- Das in Vergleichsbeispiel 2 beschriebene polymerisierbare Dentalmaterial (Initiator-Zusammensetzung mit dem wasserlöslichen Reduktionsmittel vergleichbar mit Example 5, Seite 17, aus EP 2 554 154 A1) zeigt insbesondere in Verbindung mit Bonding II einen gegenüber dem polymerisierbaren Dentalmaterial aus Vergleichsbeispiel 1 deutlich erhöhten Haftverbund. Der Haftverbund mit Bonding I liegt in diesem Fall deutlich niedriger als mit Bonding II. Hieraus lassen sich zwei Rückschlüsse ableiten: a) Der Einsatz einer wasserlöslichen reduzierenden Verbindung in dem härtbaren Dentalmaterial führt bei hydrophoben Zusammensetzungen, die für Stumpfaufbaumaterialien geeignet sind im Gegensatz zu den hydrophilen Haftmitteln aus EP 1 780 223 B1 nicht zu dem gewünschten Erfolg. b) Wird Bonding II mit einem reduzierenden Amin gemäß der Erfindung in EP 2 554 154 A1 eingesetzt, so wird durch den Einsatz einer wasserlöslichen reduzierenden Verbindung in der härtbaren Kompositzusammensetzung die Haftkraft deutlich verbessert. Dies bestätigt die in EP 2 554 154 A1 dargelegte Erfindung mit einem ein Amin als Reduktionsmittel enthaltenden speziellen Adhäsiv. Der dort gegenüber dem früheren Stand der Technik dargelegte Vorteil wird aber eindeutig nur durch die Verwendung des in dem Kit enthaltenen speziellen Adhäsives erreicht.
- Das in Vergleichsbeispiel 3 beschriebene polymere Dentalmaterial (enthält den nicht erfindungsgemäßen Phasentransferkatalysator Tetraethylammonium *p*-toluolsulfonat) zeigt mit Bonding I eine vergleichbare Haftkraft wie die Zusammensetzung von Vergleichsbeispiel 2, bei der kein Phasentransferkatalysator eingesetzt wurde. Dies bestätigt, das Phasentransferkatalysatoren mit relativ hydrophoben Anionen mit mehr als 4 C-Atomen unwirksam sind.
- Die in den Patentbeispielen 1 bis 15 beschriebenen erfindungsgemäßen polymerisierbaren Dentalmaterialien zeigen mit Bonding I einen hohen Haftverbund von mehr als 10 MPa. Dies bestätigt, dass durch den Einsatz der erfindungsgemäßen Phasentransferkatalysatoren der Haftverbund erheblich verbessert werden kann, wobei unterschiedlich substituierte Ammoniumkationen, verschiedene Anionen mit nicht mehr als 4 C-Atomen und verschiedene Konzentrationen des Phasentransferkatalysators eingesetzt werden können. Ferner sind neben den auf Ammoniumsalzen basierende Phasentransferkatalysatoren auch auf heterozyklischen Ammoniumderivaten wie dem in Patentbeispiel 12 verwendeten Imidazoliumderivat, Phosphoniumsalzen (Patentbeispiel 13 und 14) und auch Sulfoniumsalzen (Patentbeispiel 15) basierende Phasentransferkatalysatoren geeignet. Die verwendeten polymerisierbaren Dentalmaterialen weisen eine gute Lagerungsfähigkeit auf, nach einer Lagerung von 15 Monaten konnte keine Verringerung des Haftverbundes beobachtet werden. Mit dem nach EP 2 554 154 A1 erfindungsgemäßen Bonding II kann, wie mit dem Stumpfaufbaumaterial aus Patentbeispiel 3 gezeigt wird der Haftverbund nicht noch weiter verbessert werden, d.h. der Einsatz eines ein Amin als Reduktionsmittel enthaltenden speziellen Adhäsiv ist daher bei der Verwendung der nach dieser Patentschrift erfindungsgemäßen Phasentransferkatalysatoren in dem härtbaren Komposit nicht erforderlich.
- Das polymerisierbare Dentalmaterial aus Patentbeispiel 3 wurde zudem auch mit einigen kommerziellen lichthärtenden All-in-One Adhäsiven von unterschiedlichen Herstellern getestet. In allen Fällen konnte hier ein hoher Haftverbund erreicht werden.

Zusätzlich wurden mit kommerziell verfügbaren Stumpfaufbaumaterialien in Kombination mit verschiedenen kommerziell erhältlichen Adhäsiven Microtensile-Tests durchgeführt.

Für diesen Test wurden 300 intakte, kariesfreie, nicht wurzelgefüllte menschliche dritte Molare in einer wässrigen Lösung von 0.5% Chloramine T bei 4°C für maximal 30 Tage gelagert. Die Zähne wurden von Resten von Plaque und Zahnstein befreit und unter einem Lichtmikroskop mit einer 20 fachen Vergrößerung untersucht, um sicherzustellen, dass sie keine Defekte aufwiesen. Standardisierte Klasse I Kavitäten (4mm breit und lang, 4mm tief) wurden präpariert. Die Kavitäten wurden unter Verwendung von groben Diamantbohrern mit intensiver Wasserkühlung erstellt (80 µm, Two-Striper® Prep-Set, Premier, St. Paul, USA), und mit einem 25 µm Diamanten nachbehandelt. Die inneren Kanten der Kavitäten wurden abgerundet und die Ränder wurden nicht abgeschrägt. Um eine rechtwinklige Position zwischen der adhäsiv gebundenen Verbindung und der späteren Schnittrichtung der µ-TBS Stäbchen zu erzielen wurden die Höcker 2 mm abgetragen und dann der Kavitätenboden parallel zu den abgeflachten Höckern bearbeitet.

Die Kavitäten wurden mit verschiedenen Adhäsiven behandelt und auf eine Höhe von 5 mm mit den Stumpfaufbaumaterialien in der Bulk-Technik bei erhöhter Raumtemperatur (30°C zur Simulation der Mundtemperatur) überfüllt. Die Adhäsive (separat entsprechend der Anleitung der Hersteller gehärtet) und Stumpfaufbaumaterialien wurden mit einer Bluephase Dentallampe (Ivoclar Vivadent) entsprechend der Herstellerempfehlung ausgehärtet. Die Lichtintensität wurde regelmäßig mit einem Radiometer (Demetron Research Corp, Danbury, CT, USA) überprüft um sicherzustellen, dass mindestens 1200 mW/cm² während aller Experimente erreicht wurden.

Nach 24 Stunden Wassereinlagerung bei 37°C und 2.500 Thermozyklen (5°C/55°C), wurden die peripheren Bereiche der aufgebauten/gefüllten Zähne entfernt und die übrigbleibenden Prüfkörper wurden in apikaler Richtung in Scheiben geschnitten, welche dann erneut zerteilt wurden, um die Komposit-Dentin-Stäbchen zu erhalten. Die Säge wurde in Schritten von 1 mm justiert, aufgrund der Dicke des Sägeblattes (300 µm) ergeben sich daraus Stäbchen mit einer Querschnittsfläche von 700 x 700 µm (0,5 mm²). Von den erhaltenen Stäbchen wurden von jeder Gruppe wurden 20 ausgewählt (n=20). Diese 20 Stäbchen mussten mindestens eine Restdicke des Dentins von 2,0 ± 0,5 mm bis zur Pulpa aufweisen. Wenn mehr als 20 Stäbchen mit einer ausreichenden Dentin-Restdicke gesammelt werden konnten, erfolgte eine zufällige Auswahl der 20 Prüfkörper. Für den Fall, dass ein oder mehrere Stäbchen während der Auswahl versagten, wurde der prozentuale Anteil der vorzeitig versagenden Prüfkörper im Verhältnis zu der Gesamtanzahl der Prüfkörper protokolliert. Der selbe (oder angenäherte) Prozentsatz der 20 endgültigen Prüfkörper wurde mit 0 MPa in das Endergebnis des µ-TBS-Testes einbezogen. Die µ-TBS wurden in destilliertem Wasser für 24 Stunden bei 37°C gelagert und dann entsprechend einem bewährten Protokoll (Frankenberger R, Pashley DH, Reich SM, Lohbauer U, Petschelt A, Tay FR. Characterisation of resin-dentine interfaces by compressive cyclic loading. Biomaterials 2005;26:2043-2052) geprüft.

In der nachfolgenden Tabelle sind die Haftverbunde in MPa für verschiedene Kombinationen von Adhäsiven mit Stumpfaufbaumaterialien angegebenen.

| Stumpfaufbaumaterial | Adhäsiv | Haftverbund (MPa) |
|---|---|---|
| gemäß Patentbeispiel 3 | Adhese® Universal | 5,2 |
| gemäß Patentbeispiel 3 | All-Bond Universal® | 7,4 |
| gemäß Patentbeispiel 3 | Clearfill® S³ Bond Plus | 20,3 |
| gemäß Patentbeispiel 3 | Clearfill® SE Bond | 21,3 |
| gemäß Patentbeispiel 3 | Futurabond® U | 5,2 |
| gemäß Patentbeispiel 3 | i-Bond® | 5,5 |
| gemäß Patentbeispiel 3 | Scotchbond® Universal | 9,4 |
| gemäß Patentbeispiel 3 | Xeno® Select | 11,2 |
| MultiCore® Flow | Adhese® Universal | 0 |
| MultiCore® Flow | All-Bond Universal® | 0 |
| MultiCore® Flow | Clearfill® S³ Bond Plus | 0 |
| MultiCore® Flow | Clearfill® SE Bond | 0 |
| MultiCore® Flow | Futurabond® U | 0 |
| MultiCore® Flow | i-Bond® | 0 |
| MultiCore® Flow | Scotchbond® Universal | 0 |
| MultiCore® Flow | Xeno® Select | 0 |
| Rebilda DC | Adhese® Universal | 0 |
| Rebilda DC | All-Bond Universal | 0 |
| Rebilda DC | Clearfill® S³ Bond Plus | 0 |
| Rebilda DC | Clearfill® SE Bond | 0 |
| Rebilda DC | Futurabond® U | 0 |
| Rebilda DC | i-Bond® | 0 |
| Rebilda DC | Scotchbond® Universal | 0 |
| Rebilda DC | Xeno® Select | 0 |
| LuxaCore Dual | Adhese® Universal | 0 |
| LuxaCore Dual | All-Bond Universal® | 0 |
| LuxaCore Dual | Clearfill® S³ Bond Plus | 0 |
| LuxaCore Dual | Clearfill® SE Bond | 0 |
| LuxaCore Dual | Futurabond® U | 0 |
| LuxaCore Dual | i-Bond® | 0 |
| LuxaCore Dual | Scotchbond® Universal | 0 |
| LuxaCore Dual | Xeno® Select | 0 |
| Core Paste® XP | Adhese® Universal | 0 |
| Core Paste® XP | All-Bond Universal | 0 |
| Core Paste® XP | Clearfill® S³ Bond Plus | 0 |
| Core Paste® XP | Clearfill® SE Bond | 0 |
| Core Paste® XP | Futurabond® U | 0 |
| Core Paste® XP | i-Bond® | 0 |
| Core Paste® XP | Scotchbond® Universal | 0 |
| Core Paste® XP | Xeno® Select | 0 |

## Patentansprüche

1. Polymerisierbares Dentalmaterial, enthaltend eine Katalysatorpaste (A) und eine Basispaste (B), wobei die Katalysatorpaste (A) wenigstens eine organische Per-Sauerstoffverbindung, wenigstens ein radikalisch polymerisierbares organisches (Meth)acrylmonomer und wenigstens einen Füllstoff enthält, und wobei die Basispaste (B) wenigstens ein radikalisch polymerisierbares organisches (Meth)acrylmonomer, ein Amin als Co-Initiator der radikalischen Polymerisation, wenigstens einen Füllstoff und wenigstens ein darin dispergiertes salzartiges, wasserlösliches und pulverförmiges Reduktionsmittel enthält, **dadurch gekennzeichnet, dass** die Katalysatorpaste (A) und/oder die Basispaste (B) wenigstens einen Phasentransferkatalysator enthält, der ausgewählt wird aus der Gruppe der Ammonium-, Phosphonium- und/oder Sulfoniumsalze, die ein anorganisches oder organisches Anion enthalten, mit der Massgabe, dass der Phasentransferkatalysator im Fall von organischen Anionen nur solche mit 1-4 Kohlenstoffatomen aufweist und dass Anionen der Sulfinsäuren ausgenommen sind.

2. Polymerisierbares Dentalmaterial nach Anspruch 1, **dadurch gekennzeichnet, dass** der Phasentransferkatalysator nur in der Katalysatorpaste (A) vorliegt.

3. Polymerisierbares Dentalmaterial nach einem der vorhergehenden Ansprüche, **dadurch gekennzeichnet, dass** das wenigstens eine salzartige, wasserlösliche und pulverförmige Reduktionsmittel ausgewählt ist aus der Gruppe der Sulfite.

4. Polymerisierbares Dentalmaterial nach einem der vorhergehenden Ansprüche, **dadurch gekennzeichnet, dass** der Anteil des wenigstens einen Phasentransferkatalysators, bezogen auf die Gesamtmasse der Katalysatorpaste (A) und/oder der Basispaste (B), 0,01 bis 5 Gew.-% beträgt.

5. Polymerisierbares Dentalmaterial nach einem der vorhergehenden Ansprüche, **dadurch gekennzeichnet, dass** das wenigstens eine radikalisch polymerisierbare organische (Meth)acrylmonomer ausgewählt ist aus der Gruppe der aromatische Gruppen enthaltenden Acrylate oder Methacrylate, aliphatische Gruppen enthaltenden Acrylate oder Methacrylate, Polyethergruppen enthaltenden Acrylate oder Methacrylate, Polyestergruppen enthaltenden Acrylate oder Methacrylate, Polyurethangruppen enthaltende Acrylate oder Methacrylate oder Kombinationen von zwei oder mehreren dieser Monomere.

6. Polymerisierbares Dentalmaterial nach einem der vorhergehenden Ansprüche, **dadurch gekennzeichnet, dass** die radikalisch polymerisierbaren organischen (Meth)acrylmonomere frei von Struktureinheiten mit aromatischen Resten sind.

7. Polymerisierbares Dentalmaterial nach einem der vorhergehenden Ansprüche, **dadurch gekennzeichnet, dass** das Anion des Phasentransferkatalysators ausgewählt wird aus der Gruppe der Halogenide, der Hydroxide, der Anionen von anorganischen Säuren, der Pseudohalogenanionen oder der Halogenkomplexe von Aluminat, Silikat oder Phosphat, oder der Anionen von organischen Säuren mit 1-4 Kohlenstoffatomen, ausgenommen Anionen von Sulfinsäuren.

8. Polymerisiebares Dentalmaterial nach Anspruch 7, **dadurch gekennzeichnet, dass** das Anion des Phasentransferkatalysatos ausgewählt wird aus der Gruppe Fluorid, Chlorid, Bromid, lodid, Hydroxid, Sulfat, Hydrogensulfat, Dihydrogensulfat, Phosphat, Phosphonat, Borat, Chlorat, Perchlorat, Nitrit, Nitrat, Hydrogencarbonat, Carbonat, Tetrafluoroborat, Tetrachloroaluminat, Hexafluorosilikat, Hexachlorophosphat, Formiat, Acetat, Butyrat, Fumarat, Maleat, Glutarat, Lactat, Malat, Malonat, Oxalat, Pyruvat oder Tartrat.

9. Polymerisierbares Dentalmaterial nach einem der vorhergehenden Ansprüche, **dadurch gekennzeichnet, dass** der Phasentransferkatalysator ein Ammoniumsalz ist mit dem Kation NR₁R₂R₃R₄, wobei R₁, R₂, R₃ und R₄ unabhängig voneinander C₁ bis C₂₀-Alkyl, C₁ bis C₂₀-Alkylhalogenid, C₁ bis C₂₀-Alkyloxy, C₁ bis C₂₀-Hydroxyalkyl, C₂ bis C₂₀-Alkenyl, C₂ bis C₂₀-Alkenyloxy, C₂ bis C₂₀-Alkinyl, C₂ bis C₂₀-Alkinyloxy, C₂ bis C₂₀-Alkylester, Aryl, Aryloxy, Aralkyl, Aralkyloxy, Alkylaryl und/oder Alkylaryloxy bedeuten.

10. Polymerisierbares Dentalmaterial nach einem der Ansprüche 1 bis 9, **dadurch gekennzeichnet, dass** der Phasentransferkatalysator ein Phosphoniumsalz ist mit dem Kation PR₁R₂R₃R₄, wobei R₁, R₂, R₃ und R₄ unabhängig voneinander C₁ bis C₂₀-Alkyl, C₁ bis C₂₀-Alkylhalogenid, C₁ bis C₂₀-Alkyloxy, C₂ bis C₂₀-Alkenyl, C₂ bis C₂₀-Alkenyloxy, C₂ bis C₂₀-Alkinyl, C₂ bis C₂₀-Alkinyloxy, C₂ bis C₂₀-Alkylester, Aryl, Aryloxy, Aralkyl, Aralkyloxy, Alkylaryl und/oder Alkylaryloxy bedeuten.

11. Polymerisierbares Dentalmaterial nach einem der Ansprüche 1 bis 10, **dadurch gekennzeichnet, dass** der Phasentransferkatalysator ein Sulfoniumsalz ist mit dem Kation SR₁R₂R₃, wobei R₁, R₂ und R₃ unabhängig voneinander C₁ bis C₂₀-Alkyl, C₁ bis C₂₀-Alkylhalogenid, C₁ bis C₂₀-Alkyloxy, C₂ bis C₂₀-Alkenyl, C₂ bis C₂₀-Alkenyloxy, C₂ bis C₂₀-Alkinyl, C₂ bis C₂₀-Alkinyloxy, C₂ bis C₂₀-Alkylester, Aryl, Aryloxy, Aralkyl, Aralkyloxy, Alkylaryl und/oder Alkylaryloxy bedeuten.

12. Polymerisierbares Dentalmaterial nach einem der vorhergehenden Ansprüche, **dadurch gekennzeichnet, dass** der Phasentransferkatalysator Tetrabutylammoniumhydrogensulfat, Tetrahexylammoniumhydrogensulfat, Tetramethylammoniumhydrogensulfat, Tetraphenylphosphoniumchlorid, Tetrabutylphosphoniumchlorid, Bis-[tetrakis(hydroxymethyl)phosphonium]-sulfat, 1,2,3-Trimethylimidazoliummethylsulfat, 1,2,3-Trimethylimidazoliumethylsulfat, 1-Ethyl,2, 3-dimethyl-imidazoliumethylsulfat, Cetyltrimethylammoniumhydrogensulfat, (Vinylbenzyl)trimethylammoniumchlorid, Tetrabutylammoniumchlorid, Tetrabutylammoniumacetat, Tetrabutylammoniumhexafluorophosphatund/oder Tetrabutylammoniumtetrafluoroborat.

13. Polymerisierbares Dentalmaterial nach einem der vorhergehenden Ansprüche, **dadurch gekennzeichnet, dass** ein Mehrkomponentensystem enthaltend eine in einer Primärverpackung abgefüllte Katalysatorpaste (A) und eine in einer Primärverpackung abgefüllte Basispaste (B) bei einer Lagerung bei 23 °C eine Lagerstabilität von mindestens 15 Monaten aufweist.

14. Gehärtetes Dentalmaterial, erhältlich durch Vermischen der Komponenten A und B gemäß einem der Ansprüche 1 bis 13 im Verhältnis 1:20 bis 1:1 und durch Polymerisation des polymerisierbaren Dentalmaterials.

15. Verbund aus Dentin, Dentaladhäsiv und gehärtetem Material erhältlich durch Härtung des polymerisierbaren Dentalmaterials nach einem der Ansprüche 1 bis 13 **dadurch gekennzeichnet, dass** der Verbund nach 24-stündiger Lagerung bei 37 °C in Wasser und nach 2500 Thermozyklen zwischen 5 und 55 °C noch besteht.

16. Verwendung des gehärteten Dentalmaterials nach Anspruch 14 als Stumpfaufbaumaterial, als Zement-Komposit und/oder als Bulk-Fill Komposit.

17. Verwendung eines polymerisierbaren Dentalmaterials nach einem der Ansprüche 1 bis 13 zur Herstellung eines Stumpfaufbaumaterials, eines polymerisierbaren Kompositzements und/oder eines Bulk-Fill Komposits für die Herstellung von Stumpfaufbauten, Befestigungen und/oder Zahnfüllungen.

18. Baukastensystem (kit-of-parts) umfassend polymerisierbares Dentalmaterial nach einem der Ansprüche 1 bis 13 und ein Dentaladhäsiv.

## Claims

1. A polymerizable dental material, containing a catalyst paste (A) and a base paste (B), wherein the catalyst paste (A) contains at least one organic peroxygen compound, at least one radically polymerizable organic (meth)acrylic monomer and at least one filler, and wherein the base paste (B) contains at least one radically polymerizable organic (meth)acrylic monomer, an amine as co-initiator of the radical polymerization, at least one filler and at least one salt-like, water-soluble and powdery reduction agent that is dispersed therein, **characterized in that** the catalyst paste (A) and/or the base paste (B) contains at least one phase transfer catalyst that is selected from the group consisting of ammonium, phosphonium and/or sulfonium salts that contain an inorganic or organic anion, provided that the phase transfer catalyst - in the case of organic anions - contains only those having 1-4 carbon atoms, and that the anions of the sulfinic acids are precluded.

2. The polymerizable dental material according to claim 1, **characterized in that** the phase transfer catalyst is present only in the catalyst paste (A).

3. The polymerizable dental material according to any one of the preceding claims, **characterized in that** the at least one, salt-like, water-soluble and powdery reduction agent is selected from the group consisting of the sulfites.

4. The polymerizable dental material according to any one of the preceding claims, **characterized in that** the proportion of the at least one phase transfer catalyst is 0.01 to 5 percent by weight relative to the total mass of the catalyst paste (A) and/or the base paste (B).

5. The polymerizable dental material according to any one of the preceding claims, **characterized in that** the at least one radically polymerizable organic (meth)acrylic monomer is selected from the group consisting of acrylates or methacrylates containing aromatic groups, acrylates or methacrylates containing aliphatic groups, acrylates or methacrylates containing polyether groups, acrylates or methacrylates containing polyester groups, acrylates or methacrylates containing polyurethane groups or combinations of two or more of these monomers

6. The polymerizable dental material according to any one of the preceding claims, **characterized in that** the radically polymerizable organic (meth)acrylic monomers are free of any structural units having aromatic residues.

7. The polymerizable dental material according to any one of the preceding claims, **characterized in that** the anion of the phase transfer catalyst is selected from the group consisting of the halogenides, the hydroxides, the anions of inorganic acids, the pseudo halogenide anions or the halogen complexes of aluminate, silicate or phosphate, or the anions of organic acids having 1-4 carbon atoms, excluding anions of sulfinic acids.

8. The polymerizable dental material according to claim 7, **characterized in that** the anion of the phase transfer catalyst is selected from the group consisting of fluoride, chloride, bromide, iodide, hydroxide, sulfate, hydrogen sulfate, dihydrogen sulfate, phosphate, phosphonate, borate, chlorate, perchlorate, nitrite, nitrate, hydrogen carbonate, carbonate, tetrafluoroborate, tetrachloroaluminate, hexafluorosilicate, hexa-chlorophosphate, formate, acetate, butyrate, fumarate, maleate, glutarate, lactate, malate, malonate, oxalate, pyruvate or tartrate.

9. The polymerizable dental material according to any one of the preceding claims, **characterized in that** the phase transfer catalyst is an ammonium salt having the cation NR₁R₂R₃R₄, wherein R₁, R₂, R₃ and R₄ - independent of each other - mean C₁ to C₂₀ alkyl, C₁ to C₂₀ alkyl halogenide, C₁ to C₂₀ alkyloxy, C₁ to C₂₀ hydroxyalkyl, C₂ to C₂₀ alkenyl, C₂ to C₂₀ alkenyloxy, C₂ to C₂₀ alkinyl, C₂ to C₂₀ alkinyloxy, C₂ to C₂₀ alkyl ester, aryl, aryloxy, aralkyl, aralkyloxy, alkylaryl and/or alkylaryloxy.

10. The polymerizable dental material according to any of claims 1 to 9, **characterized in that** the phase transfer catalyst is a phosphonium salt having the cation PR₁R₂R₃R₄, wherein R₁, R₂, R₃ and R₄ - independent of each other - mean C₁ to C₂₀ alkyl, C₁ to C₂₀ alkyl halogenide, C₁ to C₂₀ alkyloxy, C₂ to C₂₀ alkenyl, C₂ to C₂₀ alkenyloxy, C₂ to C₂₀ alkinyl, C₂ to C₂oalkinyloxy, C₂ to C₂₀ alkyl ester, aryl, aryloxy, aralkyl, aralkyloxy, alkylaryl and/or alkylaryloxy.

11. The polymerizable dental material according to any of claims 1 to 10, **characterized in that** the phase transfer catalyst is a sulfonium salt having the cation SR₁R₂R₃, wherein R₁, R₂ und R₃ - independent of each other - mean C₁ to C₂₀ alkyl, C₁ to C₂₀ alkyl halogenide, C₁ to C₂₀ alkyloxy, C₂ to C₂₀alkenyl, C₂ to C₂₀ alkenyloxy, C₂ to C₂₀ alkinyl, C₂ to C₂₀ alkinyloxy, C₂ to C₂₀ alkyl ester, aryl, aryloxy, aralkyl, aralkyloxy, alkylaryl and/or alkylaryloxy.

12. The polymerizable dental material according to any of the preceding claims, **characterized in that** the phase transfer catalyst is tetrabutylammonium hydrogen sulfate, tetrahexylammonium hydrogen sulfate, tetramethylammonium hydrogen sulfate, tetraphenylphosphonium chloride, tetrabutylphosphonium chloride, bis-[tetrakis(hydroxymethyl)phosphonium] sulfate, 1,2,3-trimethyl-imidazolium methylsulfate, 1,2,3-trimethylimidazoliumethyl sulfate, 1-ethyl,2, 3-dimethyl-imidazolium ethylsulfate, cetyltrimethylammonium hydrogen sulfate, (vinylbenzyl)trimethylammonium chloride, tetrabutylammonium chloride, tetrabutylammonium acetate, tetrabutylammonium hexafluorophosphate and/or tetrabutylammonium tetrafluoroborate.

13. The polymerizable dental material according to any of the preceding claims, **characterized in that** a multi component system comprising the catalyst paste (A) filled in a primary packaging and the base paste (B) filled in a primary packaging exhibits a storage stability at 23 °C of at least 15 months.

14. A cured dental material obtainable by mixing the components A and B according to any of claims 1 to 13 at a ratio of 1:20 to 1:1 and by polymerization of the polymerizable dental material.

15. A composite comprising dentin, dental adhesive and cured dental material obtained by curing the polymerizable dental material according to any one of the claims 1 to 13, **characterized in that** the composite after 24 h of water storage at 37°C and after 2,500 thermocycles between 5°C and 55°C still exists.

16. A use of the cured dental material as recited in Claim 14 as core buildup material, as cement composite and/or as bulk fill composite..

17. The use of a polymerizable dental material according to any of claims 1 to 13 for producing a core buildup material, a polymerizable composite cement and/or a bulk fill composite for producing stump structures, mountings and/or tooth fillings

18. A kit system (kit of parts) comprising the polymerizable dental material as recited in one of Claims 1 through 13 and a dental adhesive.

## Revendications

1. Matériau dentaire polymérisable, contenant un catalyseur en pâte (A) et une pâte de base (B), le catalyseur en pâte (A) contenant au moins un composé organique peroxygène, au moins un monomère méthacrylique organique, polymérisable par radicaux, et au moins un agent de charge et la pâte de base (B) contenant au moins un monomère méthacrylique organique, polymérisable par radicaux, une amine en tant que co-initiateur de la polymérisation radicale, au moins un agent de charge et au moins un agent réducteur hydrosoluble et pulvérulent, à caractère salin, dispersé dans celui-ci, **caractérisé en ce que** le catalyseur en pâte (A) et/ou la pâte de base (B) contient au moins un catalyseur de transfert de phase, qui est choisi dans le groupe des sels d'ammonium, de phosphonium et/ou de sulfonium, qui contiennent un anion inorganique ou organique avec le critère que dans le cas d'anions organiques, le catalyseur de transfert de phase n'en contienne que de tels pourvus de 1 à 4 atomes de carbone et **en ce que** des anions des acides sulfiniques sont exceptés.

2. Matériau dentaire polymérisable selon la revendication 1, **caractérisé en ce que** le catalyseur de transfert de phase n'est présent que dans le catalyseur en pâte (A) .

3. Matériau dentaire polymérisable selon l'une quelconque des revendications précédentes, **caractérisé en ce que** l'au moins un agent réducteur hydrosoluble et pulvérulent, à caractère salin est choisi dans le groupe des sulfites.

4. Matériau dentaire polymérisable selon l'une quelconque des revendications précédentes, **caractérisé en ce que** la part de l'au moins un catalyseur de transfert de phase, rapportée à la masse totale du catalyseur en pâte (A) et/ou de la pâte de base (B) d'élève à de 0,01 à 5 % en poids.

5. Matériau dentaire polymérisable selon l'une quelconque des revendications précédentes, **caractérisé en ce que** l'au moins un monomère (méth)acrylique organique, polymérisable par radicaux est choisi dans le groupe des acrylates ou méthacrylates contenant des groupes aromatiques, des acrylates ou méthacrylates contenant des groupes aliphatiques, des acrylates ou méthacrylates contenant des groupes polyester, des acrylates ou méthacrylates contenant des groupes polyuréthane, ou des associations de deux ou plusieurs desdits monomères.

6. Matériau dentaire polymérisable selon l'une quelconque des revendications précédentes, **caractérisé en ce que** les monomères méthacryliques organiques, polymérisables par radicaux sont exempts d'unités structurelles avec des restes aromatiques.

7. Matériau dentaire polymérisable selon l'une quelconque des revendications précédentes, **caractérisé en ce que** l'anion du catalyseur de transfert de phase est choisi dans le groupe des halogénures, des hydroxydes, des anions d'acides inorganiques, des anions de pseudo-halogénures ou des complexes halogénés d'aluminate, de silicate ou de phosphate, ou des anions d'acides organiques pourvus de 1 à 4 atomes de carbone, à l'exception des anions des acides sulfiniques.

8. Matériau dentaire polymérisable selon la revendication 7, **caractérisé en ce que** l'anion du catalyseur de transfert de phase est choisi dans le groupe comprenant les fluorures, les chlorures, les bromures, les iodures, les hydroxydes, les sulfates, les sulfates d'hydrogène, les sulfates de dihydrogène, les phosphates, les phosphonates, les borates, les chlorates, les perchlorates, les nitrites, les nitrates, les carbonates d'hydrogène, les carbonates, les tétrafluoroborates, les tétrachloroaluminates, les hexafluorosilicates, les hexachlorophosphates, les formiates, les acétates, les butyrates, les fumarates, les maléates, les glutarates, les lactates, les malates, les malonates, les oxalates, les pyruvates ou les tartrates.

9. Matériau dentaire polymérisable selon l'une quelconque des revendications précédentes, **caractérisé en ce que** le catalyseur de transfert de phase est un sel d'ammonium comprenant le cation NR₁R₂R₃R₄, R₁, R₂, R₃ et R₄ étant mis, indépendamment les uns des autres pour un alkyle en C₁ à C₂₀, un halogénure d'alkyle en C₁ à C₂₀, un alkyloxy en C₁ à C₂₀, un hydroxyalkyle en C₁ à C₂₀, un alkényle en C₂ à C₂₀, un alkényloxy en C₂ à C₂₀, un alkinyle en C₂ à C₂₀, un alkinyloxy en C₂ à C₂₀, un alkylester en C₂ à C₂₀, un aryle, un aryloxy, un aralkyle, un aralkyloxy, un alkylaryle et/ou un alkylaryloxy.

10. Matériau dentaire polymérisable selon l'une quelconque des revendications 1 à 9, **caractérisé en ce que** le catalyseur de transfert de phase est un sel de phosphonium comprenant le cation PR₁R₂R₃R₄, R₁, R₂, R₃ et R₄ étant mis, indépendamment les uns des autres pour un alkyle en C₁ à C₂₀, un halogénure d'alkyle en C₁ à C₂₀, un alkyloxy en C₁ à C₂₀, un alkényle en C₂ à C₂₀, un alkényloxy en C₂ à C₂₀, un alkinyle en C₂ à C₂₀, un alkinyloxy en C₂ à C₂₀, un alkylester en C₂ à C₂₀, un aryle, un aryloxy, un aralkyle, un aralkyloxy, un alkylaryle et/ou un alkylaryloxy.

11. Matériau dentaire polymérisable selon l'une quelconque des revendications 1 à 10, **caractérisé en ce que** le catalyseur de transfert de phase est un sel de sulfonium comprenant le cation SR₁R₂R₃, R₁, R₂ et R₃ étant mis, indépendamment les uns des autres pour un alkyle en C₁ à C₂₀, un halogénure d'alkyle en C₁ à C₂₀, un alkyloxy en C₁ à C₂₀, un alkényle en C₂ à C₂₀, un alkényloxy en C₂ à C₂₀, un alkinyle en C₂ à C₂₀, un alkinyloxy en C₂ à C₂₀, un alkylester en C₂ à C₂₀, un aryle, un aryloxy, un aralkyle, un aralkyloxy, un alkylaryle et/ou un alkylaryloxy.

12. Matériau dentaire polymérisable selon l'une quelconque des revendications précédentes, **caractérisé en ce que** le catalyseur de transfert de phase est un sulfate de tétrabutylammonium hydrogéné, un sulfate de tétrahexylammonium hydrogéné, un sulfate de, tétraméthylammonium hydrogéné, un chlorure de tétraphénylphosphonium, un chlorure de tétrabutylphosphonium, un sulfate de bis-[tétrakis(hydroxyméthyl)phosphonium], un sulfate de 1,2,3-triméthylimidazoliumméthyle, un sulfate de 1-éthyl,2, 3-diméthyl-imidazoliuméthyle, un sulfate de cétyltriméthylammonium hydrogéné, un chlorure de (vinylbenzyl)triméthylammonium, un chlorure de tétrabutylammonium, un acétate de tétrabutylammonium, un hexafluorophosphate de tétrabutylammonium et/ou un tétrafluoroborate de tétrabutylammonium.

13. Matériau dentaire polymérisable selon l'une quelconque des revendications précédentes, **caractérisé en ce que** lors d'un stockage à 23° C, un système multicomposants comprenant un catalyseur en pâte (A) contenu dans un emballage primaire et une pâte de base (B) contenue dans un emballage primaire fait preuve d'une stabilité au stockage d'au moins 15 mois.

14. Matériau dentaire solidifié, susceptible d'être obtenu par mélange des composants A et B selon l'une quelconque des revendications 1 à 3 dans un rapport de 1:20 à 1:1 et par polymérisation du matériau dentaire polymérisable.

15. Composite de dentine, d'adhésif dentaire et de matériau solidifié susceptible d'être obtenu par solidification du matériau dentaire polymérisable selon l'une quelconque des revendications 1 à 13, **caractérisé en ce que** le composite reste préservé après un stockage de 24 heures à 37 °C dans de l'eau et après 2500 thermo-cycles entre 5 et 55 °C.

16. Utilisation du matériau dentaire solidifié selon la revendication 14 comme matériau de reconstitution de moignon, comme composite de ciment et/ou comme composite en vrac.

17. Utilisation d'un matériau dentaire polymérisable selon l'une quelconque des revendications 1 à 13 pour la fabrication d'un matériau de reconstitution de moignons, d'un ciment composite polymérisable et/ou d'un composite en vrac pour la fabrication de moignons, de fixations et/ou d'obturations dentaires.

18. Système modulaire (kit-of-parts) comprenant un matériau dentaire polymérisable selon l'une quelconque des revendications 1 à 13 et un adhésif dentaire.
